# EUROPEAN PATENT SPECIFICATION

(11) **EP 3 965 799 B1**
(45) Date of publication and mention of the grant of the patent: **02.12.2026**
(21) Application number: 20827319.3
(22) Date of filing: 21.06.2020
(51) Int. Cl.: A61K 38/17, A61K 45/06, A61P 31/00, A61K 31/407, A61K 31/43

(54) **COMBINATION OF GELSOLIN AND A BETA LACTAM ANTIBIOTIC FOR USE IN THE TREATMENT OF AN INFECTION CAUSED BY A RESISTANT PNEUMOCOCCAL SPECIES**
KOMBINATION AUS GELSOLIN UND EINEM BETA-LAKTAM-ANTIBIOTIKUM ZUR VERWENDUNG BEI DER BEHANDLUNG EINER INFEKTION, DIE DURCH EINE RESISTENTE PNEUMOKOKKEN-SPEZIES VERURSACHT WIRD
COMBINAISON DE GELSOLINE ET D'UN ANTIBIOTIQUE BETA-LACTAME POUR UNE UTILISATION DANS LE TRAITEMENT D'UNE INFECTION CAUSÉE PAR UNE ESPÈCE DE PNEUMOCOQUE RÉSISTANTE

(30) Priority: 21.06.2019 US 201962864599 P
(43) Date of publication of application: 16.03.2022
(73) Proprietor: BioAegis Therapeutics Inc., Morristown, NJ 07960-2700 (US); President and Fellows of Harvard College, Cambridge, MA 02138 (US)
(72) Inventor: DINUBILE, Mark J., Morristown, NJ 07960-2700 (US); LEVINSON, Susan L., Morristown, NJ 07960-2700 (US); STOSSEL, Thomas P., Morristown, NJ 07960-2700 (US); KOBZIK, Lester, San Carlos, CA 94070 (US)
(74) Representative: Barker Brettell LLP
(86) International application number: PCT/US2020/038862
(87) International publication number: WO 2020/257743

(56) References cited:
- WO-A2-2005/046454
- AU-B2- 2011 218 617
- US-A1- 2008 118 945
- US-A1- 2017 189 483
- WEINER DANIEL J ET AL: "The antimicrobial activity of the cathelicidin LL37 is inhibited by F-actin bundles and restored by gelsolin", AMERICAN JOURNAL OF RESPIRATORY CELL AND MOLECULAR BIOLOGY, AMERICAN LUNG ASSOCIATION, NEW YORK, NY, US, vol. 28, no. 6, 1 June 2003 (2003-06-01), pages 738 - 745, XP002431941, ISSN: 1044-1549, DOI: 10.1165/RCMB.2002-0191OC
- YANG ZHIPING ET AL: "Delayed Administration of Recombinant Plasma Gelsolin Improves Survival in a Murine Model of Penicillin-Susceptible and Penicillin-Resistant Pneumococcal Pneumonia", JOURNAL OF INFECTIOUS DISEASES, vol. 220, no. 9, 9 July 2019 (2019-07-09), pages 1498 - 1502, XP055830016, ISSN: 0022-1899, Retrieved from the Internet <URL:https://www.ncbi.nlm.nih.gov/pmc/articles/PMC6761947/pdf/jiz353.pdf> DOI: 10.1093/infdis/jiz353
- YANG Z. ET AL: "Delayed therapy with plasma gelsolin improves survival in murine pneumococcal pneumonia", ID WEEK 2017, vol. 4, 1 September 2017 (2017-09-01), pages S474 - S475, XP093352385, Retrieved from the Internet <URL:https://watermark02.silverchair.com/ofx163.1215.pdf?token=AQECAHi208BE49Ooan9kkhW_Ercy7Dm3ZL_9Cf3qfKAc485ysgAAA3kwggN1BgkqhkiG9w0BBwagggNmMIIDYgIBADCCA1sGCSqGSIb3DQEHATAeBglghkgBZQMEAS4wEQQMKTxfG7Q9Fqj0pvIBAgEQgIIDLAzB6B3kjk8AhhA3SkF85NzoV1sgvFM2QYDA2JfCs_Su72CUYpOKshZFE-baqQIRS2zSxuCko-EvBELgG2UfzW>
- DINUBILE MARK J ET AL: "Recombinant Human Plasma Gelsolin Improves Survival and Attenuates Lung Injury in a Murine Model of Multidrug-Resistant Pseudomonas aeruginosa Pneumonia", OPEN FORUM INFECTIOUS DISEASES, vol. 7, no. 8, 19 June 2020 (2020-06-19), pages ofaa236, XP093352396, ISSN: 2328-8957, Retrieved from the Internet <URL:https://pmc.ncbi.nlm.nih.gov/articles/PMC7397834/pdf/ofaa236.pdf> DOI: 10.1093/ofid/ofaa236
- KWIATKOWSKI D J ET AL: "PLASMA AND CYTOPLASMIC GELSOLINS ARE ENCODED BY A SINGLE GENE AND CONTAIN A DUPLICATED ACTIN-BINDING DOMAIN", NATURE,, vol. 323, 2 October 1986 (1986-10-02), pages 455 - 458, XP002911329, DOI: 10.1038/323455A0
- T.-C. CHOU: "Drug Combination Studies and Their Synergy Quantification Using the Chou-Talalay Method", CANCER RESEARCH, vol. 70, no. 2, 15 January 2010 (2010-01-15), pages 440 - 446, XP055169871, ISSN: 0008-5472, DOI: 10.1158/0008-5472.CAN-09-1947
- YANG ZHIPING ET AL: "Plasma gelsolin improves lung host defense against pneumonia by enhancing macrophage NOS3 function", AMERICAN JOURNAL OF PHYSIOLOGY - LUNG CELLULAR AND MOLECULAR PHYSIOLOGY, vol. 309, no. 1, 1 July 2015 (2015-07-01), US, pages L11 - L16, XP093400784, ISSN: 1040-0605, DOI: 10.1152/ajplung.00094.2015
- COHEN TAYLOR S. ET AL: "Therapeutic potential of plasma gelsolin administration in a rat model of sepsis", CYTOKINE, vol. 54, no. 3, 1 June 2011 (2011-06-01), US, pages 235 - 238, XP093400807, ISSN: 1043-4666, Retrieved from the Internet <URL:https://pdf.sciencedirectassets.com/272515/1-s2.0-S1043466611X00054/1-s2.0-S1043466611000354/main.pdf?hash=338e763a56e9fbd6ffb14b2489f6c655ab11b62dcce98a717eb99d15d3b8d93b&host=68042c943591013ac2b2430a89b270f6af2c76d8dfd086a07176afe7c76c2c61&pii=S1043466611000354&tid=spdf-bf2d3422-2a2c-4eeb-b380-7b3> DOI: 10.1016/j.cyto.2011.02.006
- LEE P -S ET AL: "Plasma gelsolin is a marker and therapeutic agent in animal sepsis", CRITICAL CARE MEDICINE LIPPINCOTT WILLIAMS & WILKINS, vol. 35, no. 3, 1 March 2007 (2007-03-01), pages 849 - 855, XP008122402, ISSN: 0090-3493, DOI: 10.1097/01.CCM.0000253815.26311.24
- BALA K. ET AL: "Antibiotic Resistance - StatPearls - NCBI Bookshelf", 31 January 2026 (2026-01-31), pages 1 - 10, XP093400932, Retrieved from the Internet <URL:https://www.ncbi.nlm.nih.gov/books/NBK513277/>
- BRADLEY JOHN S. ET AL: "The Management of Community-Acquired Pneumonia in Infants and Children Older Than 3 Months of Age: Clinical Practice Guidelines by the Pediatric Infectious Diseases Society and the Infectious Diseases Society of America", CLINICAL INFECTIOUS DISEASES, vol. 53, no. 7, 1 October 2011 (2011-10-01), pages e25 - e76, XP093406567, ISSN: 1058-4838, DOI: 10.1093/cid/cir531

## Description

### Field of the Invention

The invention relates to pharmaceutical compositions for use in the treatment of a bacterial infection resistant to an antibacterial agent in a subject.

### Background of the Invention

Antimicrobial resistance is a worldwide public health concern. Antimicrobial resistance is known to reduce therapeutic efficacy of a variety of antimicrobial agents such as antibiotic agents, antiviral agents, antifungal agents, and antiparasitic agents. Examples of the evolving presence of resistant pneumococcal bacterial strains include: a case report of fatal resistant pneumococcal pneumonia (Waterer GW et al., Chest 2000; 118: 1839-1840) and a finding that 22% (139/643) patients hospitalized for *S. pneumonia* had macrolideresistant organisms (Cilloniz et al., Am J Respir Crit Care Med 2015; 191: 1265-1272). Recent publications evidence (1) resistance of *S. pneumoniae* isolates from invasive infections to erythromycin (96%), trimethoprim-sulfamethoxazole (79%) and tetracycline (77%) in a pediatric population (Cai et al., Infect Drug Resist 2018; 11: 2461-2469) and (2) a survey of *S. pneumoniae* isolates from invasive infections in an older population (Intra et al., Front Public Health 2017; 5: 169). A review publication, Kollef & Betthauser, Curr. Opin. Inf. Dis. 2019; 32: 169-175, emphasizes increasing antibiotic resistance in common bacterial pathogens associated with community-acquired pneumonia (CAP), especially staphylococci and *Streptococcus pneumonia.*

Antimicrobial agents have long been used to treat microbial infections because of their therapeutic effects against microbial infections in humans and animals. Resistance to a previously therapeutically effective antimicrobial agent may be caused by a change in the infection-causing pathogen. Overuse and misuse of antimicrobials may be a factor in the growing problem of antimicrobial resistance, which continues to result in increasing types of pathogenic infections that are less responsive to previously effective antimicrobial agents. Antimicrobial resistance results in a lack of therapeutic options with which to treat pathogenic infections. Antimicrobial-resistant pathogens result in numerous deaths each year and are a serious worldwide public health challenge.

Yang Z. et al: "Delayed therapy with plasma gelsolin improves survival in murine pneumococcal pneumonia". ID Week 2017, vol. 4, 1 September 2017, pages S474-S475 discloses that rhu-pGSN can improve survival in a murine model of fatal pneumococcal pneumonia, even when administered as single doses on days 2 and 3 after infection without antibiotics.

### Summary of the Invention

The scope of protection is defined in the appended claims. Specifically, the invention provides a pharmaceutical composition for use in a method of treatment of a bacterial infection resistant to an antibacterial agent in a subject, wherein the bacterial infection is caused by a Pneumococcal species, and wherein:
(a) the pharmaceutical composition comprises a gelsolin molecule, the method comprising administering the pharmaceutical composition to the subject and administering the antibacterial agent to which the bacterial infection is resistant to the subject, to thereby treat the bacterial infection, wherein the gelsolin molecule and the antibacterial agent are administered in the same or separate formulations, but are administered to be in the subject at the same time; or
(b) the pharmaceutical composition comprises the antibacterial agent to which the bacterial infection is resistant, the method comprising administering the pharmaceutical composition to the subject and administering a gelsolin molecule to the subject, to thereby treat the bacterial infection wherein the gelsolin molecule and the antibacterial agent are administered in the same or separate formulations, but are administered to be in the subject at the same time; or
(c) the pharmaceutical composition comprises a gelsolin molecule and the antibacterial agent to which the bacterial infection is resistant, the method comprising administering the pharmaceutical composition to the subject, to thereby treat the bacterial infection;
wherein the gelsolin molecule is a plasma gelsolin (pGSN) or a cytoplasmic gelsolin (cGSN), and wherein the antibacterial agent comprises a β-lactam antibiotic.

No claim is made to methods of medical treatment. Any reference to a method of treatment should be understood as being in the context of the claimed pharmaceutical composition for use in a method of treatment.

In some embodiments, the antibacterial agent is in a clinically acceptable amount and the administered gelsolin molecule and antibacterial agent synergistically enhance a therapeutic effect of administering the clinically acceptable amount of the antibacterial agent and not the gelsolin molecule to the subject. In certain embodiments, the clinically acceptable amount of the antibacterial agent is an amount below a maximum tolerated dose (MTD) of the antibacterial agent in the subject. In some embodiments, the MTD of the antibacterial agent is a highest possible but still tolerable dose level of the antibacterial agent for the subject. In some embodiments, the MTD of the antibacterial agent is determined at least in part on a pre-selected clinical-limiting toxicity for the antibacterial agent in the subject. In certain embodiments, the synergistically effective amount of the gelsolin molecule and the antibacterial agent decreases a minimum effective dose (MED) of the antibacterial agent in the subject. In certain embodiments, the MED is a lowest dose level of the antibacterial agent that provides a clinically significant response in average efficacy, wherein the response is statistically significantly greater than a response provided by a control that does not include the dose of the antibacterial agent. In some embodiments, the synergistic therapeutic effect of the gelsolin molecule and the antibacterial agent includes increasing a likelihood of survival of the subject. In some embodiments, the synergistic therapeutic effect of the gelsolin molecule and the antibacterial agent includes reducing the bacterial infection in the subject.. In some embodiments, the antibacterial agent includes penicillin. In some embodiments, the antibacterial agent is meropenem. In some embodiments, the subject is a mammal, optionally a human. In some embodiments, the gelsolin molecule comprises plasma gelsolin (pGSN), and optionally is a recombinant pGSN. In some embodiments, the composition also includes a pharmaceutically acceptable carrier. In some embodiments, the composition also includes a pharmaceutically acceptable carrier.

In some embodiments, the level of the bacterial infection in the subject is determined, and a means of the determining comprises one or more of: an assay, observing the subject, assessing one or more physiological symptoms of the bacterial infection in the subject, and assessing a likelihood of survival of the subject. In some embodiments, the physiological symptoms comprise one or more of: fever, malaise, and death. In certain embodiments, the physiological symptoms comprise lung pathology. In some embodiments, the physiological symptoms comprise weight loss. In some embodiments, the assay comprises a means for detecting the presence, absence, and/or level of a characteristic of the bacterial infection in a biological sample from the subject. In some embodiments, the administration of the synergistically effective amount of each of the antibacterial agent and the gelsolin molecule increases the subject's likelihood of survival compared to a control likelihood of survival. In certain embodiments, the control likelihood of survival is a likelihood of survival in the absence of the administration of the synergistically effective amount of each of the antibacterial agent and the gelsolin molecule. In some embodiments, the increase in the subject's likelihood of survival is at least 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, or 200% higher than the control likelihood of survival. In certain embodiments, the administration of the synergistically effective amount of each of the antibacterial agent and the gelsolin molecule reduces a level of lung pathology in the subject compared to a control level of lung pathology. In some embodiments, the control level of lung pathology is a level of lung pathology in the absence of the administration of the synergistically effective amount of each of the antibacterial agent and the gelsolin molecule. In certain embodiments, the level of lung pathology in the subject administered the synergistically effective amount of each of the antibacterial agent and the gelsolin molecule is at least 5%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, or 200% lower than the control level of lung pathology. In some embodiments, the administration means of the gelsolin molecule and the antibacterial agent are independently selected from: oral, sublingual, buccal, intranasal, intravenous, intramuscular, intrathecal, intraperitoneal, subcutaneous, intradermal, topical, rectal, vaginal, intrasynovial, and intra-ocular administration. In some embodiments, the subject is a mammal, and optionally is a human. In some embodiments, the gelsolin molecule and the antibacterial agent are administered to a subject separately or simultaneously.

### Brief Description of the Drawings

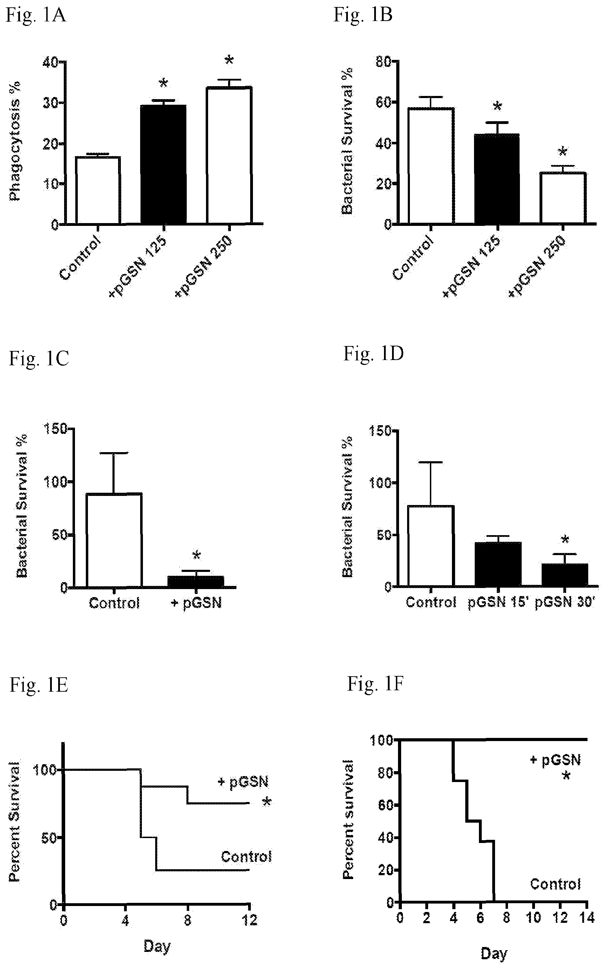

Figure 1A-F shows graphs of results of systemic experiments that measure improvements in host defense against bacterial pneumonia following administration of pGSN. *In vitro,* pGSN improves macrophage uptake (Fig. 1A) and killing of internalized pneumococci (Fig. 1B) when present at 125-250 µg/ml, similar to normal plasma levels. *In vivo,* pGSN (10 mg s.c. 2 h before and 8 and 20 h after infection improved bacterial clearance (fewer surviving bacteria at 24h) in Bl6 mice challenged with 10⁵ pneumococci by i.n. insufflation (Fig. 1C); similar results were seen when pGSN was administered as an aerosol for 15 or 30 minutes prior to infection (Fig. 1D). Systemic pGSN (s.c.) improved survival in primary (Fig. 1E, using 3 X 10⁵ CFU inoculum) or secondary post-influenza pneumococcal pneumonia (Fig. 1F, using 500 CFU inoculum on day 7 after mild influenza infection with PR8) even in the absence of any antibiotic treatment. * = p<.05 vs control, n= 6-12 per group. All experiments used serotype 3 *Strep. Pneumoniae.*

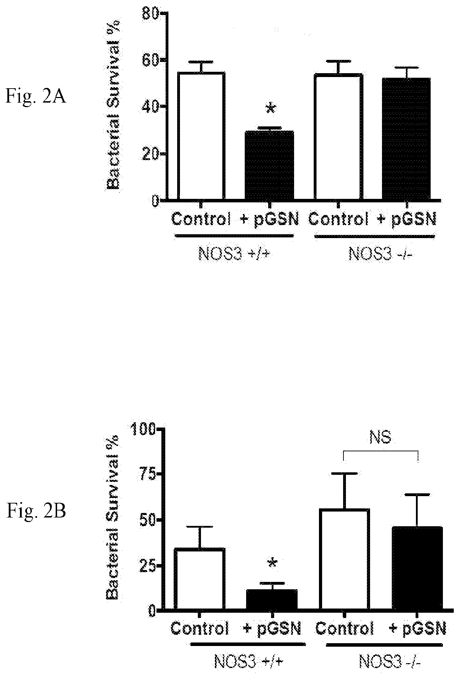

Figure 2A-B provides graphs showing that the effect of pGSN on macrophages requires NOS3. Fig. 2A shows experimental results that demonstrated macrophage killing of pneumococci *in vitro.* Fig. 2B shows experimental results indicating macrophage clearance of bacteria *in vivo.* Macrophage clearance of bacteria was no longer enhanced if NOS3-deficient cells or animals were used. * = p < .01.

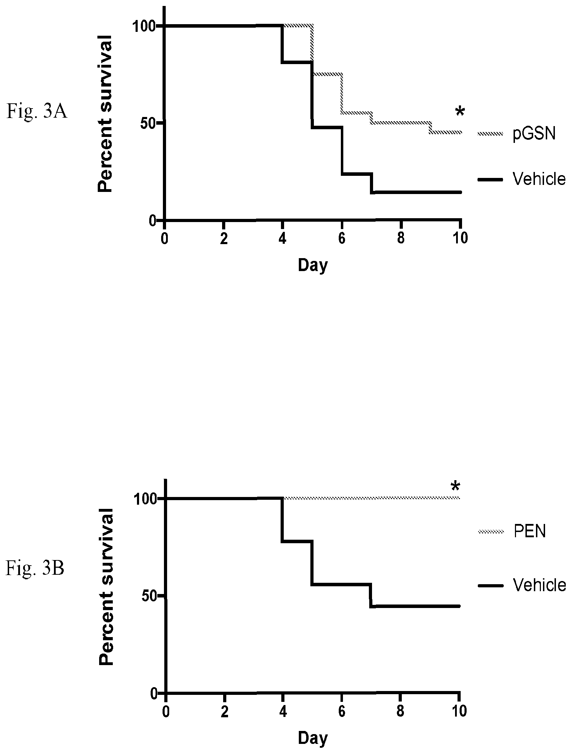

Figure 3A-B provides graphs of studies of antibiotic-sensitive pneumococcal pneumonia. Fig. 3A shows results of treatment with pGSN (5 mg i.p. on days 2 and 3 after infection) demonstrating improved survival in mice infected with serotype 3 pneumococci (*= p =.01, n= 20/group, summary of 2 trials, 10 mice per group per trial). Fig. 3B provides results of treatment with penicillin (PEN, 100 µg i.m. on days 2 and 3 after infection) indicating improved survival in mice infected with serotype 3 pneumococci (*= p =.02, n= 8-9/group, single trial).

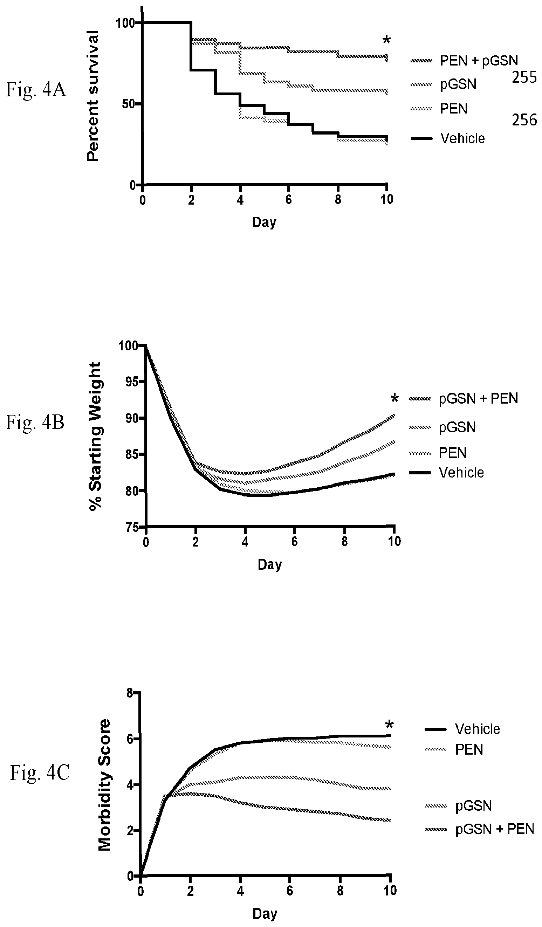

Figure 4A-C provides graphs of results of studies with antibiotic-resistant pneumococcal pneumonia. Results in Fig. 4A demonstrate treatment with pGSN (5 mg i.p. daily starting on day 1 after infection) improved survival in mice infected with serotype 14 pneumococci compared to vehicle or penicillin (PEN, 1 mg dose i.m daily) (*, p = .02, .04 respectively, log rank comparisons after Sidak adjustment for multiple comparisons). Combined treatment with pGSN and penicillin also resulted in higher survival compared to vehicle or penicillin (**, p = .0001 for both comparisons, log rank with Sidak adjustment for multiple comparisons). Survival of the pGSN vs. pGSN + PEN groups was not statistically significant after Sidak adjustment for multiple comparisons (p = .47 n= 38-41/group, summary of 4 trials, 8-11 mice per group per trial). Assessment of (Fig. 4B) weight loss and (Fig. 4C) morbidity showed more rapid recovery of weight and a lower morbidity index in the pGSN or pGSN + PEN groups (mean values for each day shown, p = .001, p = .04 respectively, ANOVA; n = 38-41 per group in 4 trials for B, n = 30 per group in 3 trials for C; the last observation for any mouse was carried forward after death).

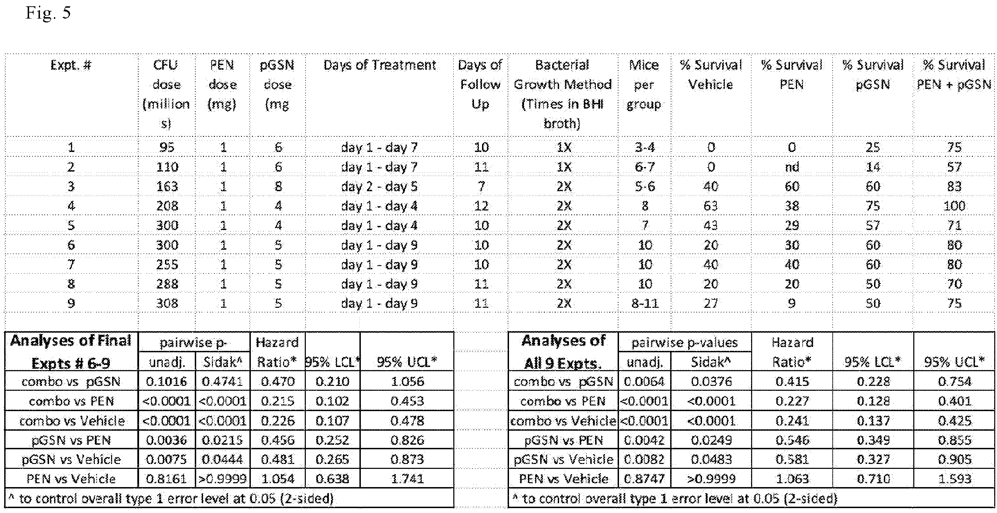

Figure 5 provides a table with data results from nine experiments in which testing delayed administration of four treatments was assessed. Fig. 5 presents details of nine experiments, including pilot and range-finding trials. Column H shows a change in bacterial growth method obtained using a method for 2X growth in BHI broth for penicillin-resistant pneumococci [Restrepo AV et al., BMC Microbiol 2005; 5:34] for superior growth results. The data provided in the table demonstrate that in all nine experiments, survival was highest in the PEN + pGSN group. Survival in the PEN + pGSN group was higher than in the pGSN group, and survival in both were higher than in the vehicle or PEN alone groups. The survival differences were statistically significant, as determined by analysis of all the nine studies pooled using log rank analysis along with Sidak correction for multiple comparisons. Details of the results of statistical analysis of the final four experiments (#6-9) are summarized in Fig. 4A-C.

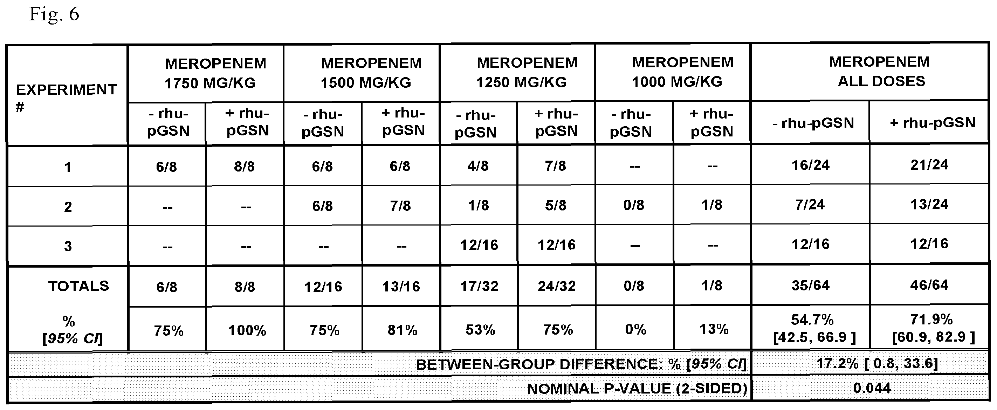

Figure 6 provides a table with data from three experiments assessing survival following administration of meropenem doses with or without rhu-pGSN to neutropenic mice. Fig. 6 presents details of three experiments in which meropenem doses as indicated were administered subcutaneously beginning at 3 h post-infection with MDR *P. aeruginosa* and q8h thereafter for 5 days. Meropenem doses were administered either with or without rhu-pGSN. rhu-pGSN was administered as 12 mg via intraperitoneal injection on Days -1, 0, 1, 2, 3, 4, and 5. n/N = number of surviving mice / Number of treated mice.

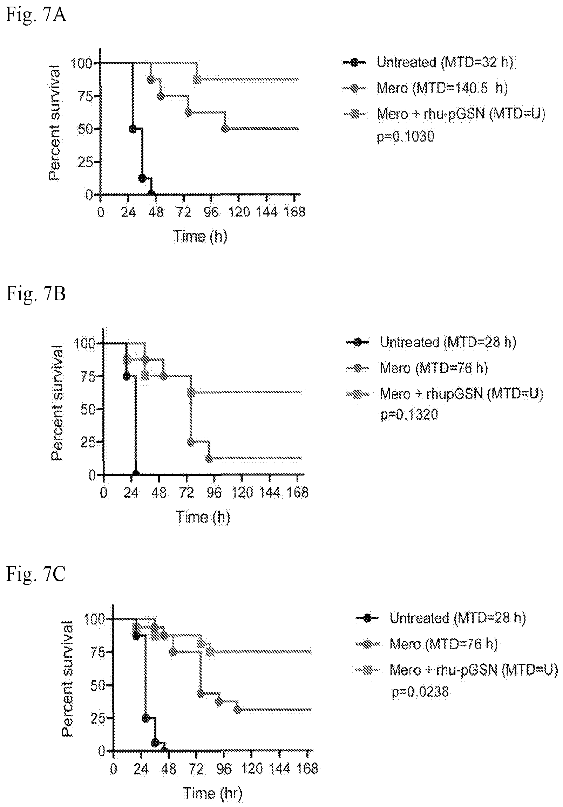

Figure 7A-C provides graphs demonstrating the survival benefit observed with combined meropenem and rhu-pGSN treatment. BALB/c- mice made neutropenic with cyclophosphamide (BALB/c-Cy mice) were infected with the UNC-D strain of *P. aeruginosa* and treated with either meropenem alone (1250 mg/kg/day subcutaneously q8h for 5 days beginning 3 h post-infection) or in combination with pGSN (12 mg/day intraperitoneally daily for days -1 to +5). Mice were euthanized upon reaching endpoint criteria or at the study conclusion on Day 7. Survival analysis was conducted by log-rank test using two studies of n = 8 group size (Fig. 7A and Fig. 7B), where the control mortality rate at Day 7 was ≥ 50% with the same 1250 mg meropenem dose. The results were then analyzed by combining these two separate studies (Fig. 7C). The p values refer to the survival advantage of combination therapy over meropenem alone. MTD, mean time to death.

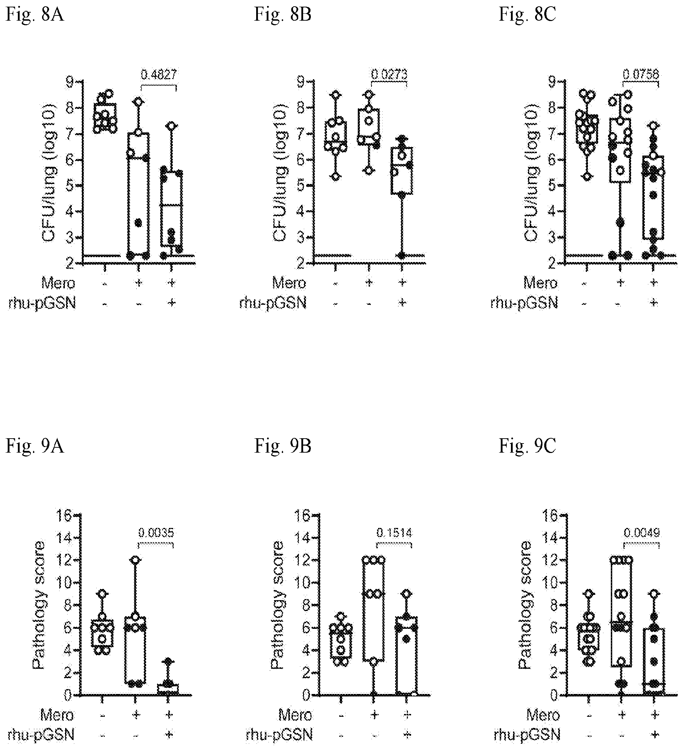

Figure 8A-C provides graphs illustrating that rhu-pGSN administration reduces bacterial counts in the lungs. Two studies were performed in which BALB/c-Cy mice were infected with the UNC-D strain of *P. aeruginosa* and treated with either meropenem alone (1250 mg/kg/day subcutaneously q8h for 5 days beginning 3 h post-infection) or in combination with pGSN (12 mg/day intraperitoneally daily for days -1 to +5). Mice were euthanized upon reaching endpoint criteria (open circle) or survivors at the study conclusion on Day 7 (closed circle). Bacteria were enumerated from homogenized lung by plate count. Fig. 8A shows a graph of the results of the first study; Fig. 8B shows a graph of the results from the second study. Individual and combined data were analyzed for the two studies and with pairwise analysis of meropenem therapy alone (Mero) versus in combination with pGSN. p values refer to unpaired Student t-test comparisons of combination therapy versus meropenem alone. The lines at the bottom of the graph indicate the limit of detection. Fig. 8C shows a graph of combined data from the two studies shown in Fig. 8A and Fig. 8B.
Figure 9A-C provides graphs illustrating that rhu-pGSN limits infection-induced lung injury. Two studies were performed in which BALB/c-Cy mice were infected with the UNC-D strain of *P. aeruginosa* and treated with either meropenem alone (1250 mg/kg/day subcutaneously q8h for 5 days beginning 3 h post-infection) or in combination with pGSN (12 mg/day intraperitoneally daily for days -1 to +5). Mice were euthanized upon reaching endpoint criteria (open circle) or survivors at the study conclusion on Day 7 (closed circle). A representative section of lung was excised from the lung and processed for H&E staining and scoring. Fig. 9A shows a graph of the results of the first study; Fig. 9B shows a graph of the results of the second study. Data was analyzed for the two individual studies separately and combined with pairwise analysis of meropenem therapy alone (Mero) or in combination with pGSN. The p values refer to unpaired Student t- test comparisons of combination therapy versus meropenem alone. Fig. 9C shows a graph of combined data from the two studies shown in Fig. 9A and Fig. 9B.

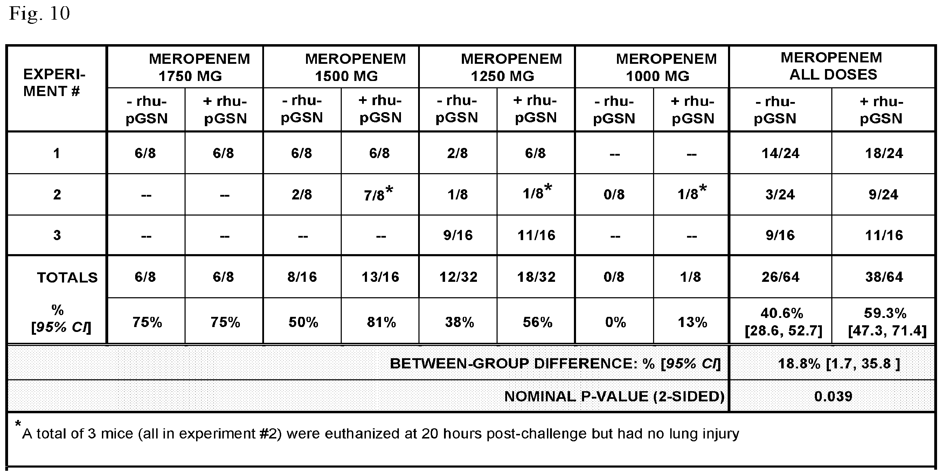

Figure 10 provides a table with data of overall survival with minor lung injury from surviving mice treated with different doses of meropenem. Meropenem doses as indicated were administered subcutaneously beginning at 3 h post-infection and q8h thereafter for 5 days. rhu-pGSN was administered as 12 mg via intraperitoneal injection on Days -1, 0, 1, 2, 3, 4, and 5. Asterisk (*) indicates that a total of 3 mice (all in experiment #2) were euthanized at 20 hours post-challenge but had no lung injury; there was one mouse in each of the three meropenem + rhu-pGSN treatment groups. Excluding these 3 mice from the rhu-pGSN tallies yielded a final count of 41/61 (67.2%). n/N = number of surviving mice with composite Lung Injury Scores ≤ 2 / Number of treated mice.

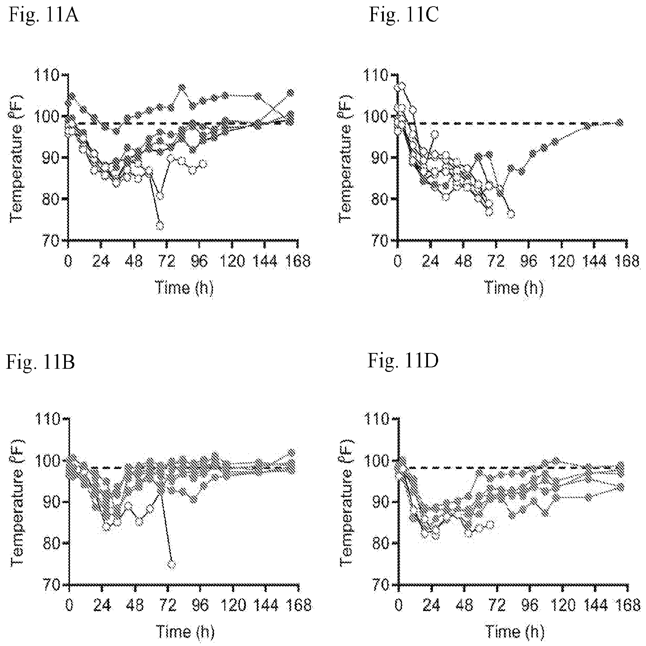

Figure 11A-D presents graphs from two experiments demonstrating restoration of baseline temperature in mice treated with either meropenem alone or with meropenem plus rhu-pGSN. In both experiments, BALB/c-Cy mice were infected with the UNC-D strain of *P. aeruginosa.* Fig. 11A shows a graph of temperatures from mice in the first experiment treated with meropenem alone (1250 mg/kg/day subcutaneously q8h for 5 days beginning 3 h post-infection); Fig. 11C shows a graph of temperatures from mice in the second experiment treated with meropenem alone (same regimen as in Fig. 11A). Fig. 11B shows a graph of temperatures from mice in the first experiment treated with meropenem in combination with rhu-pGSN (12 mg/day intraperitoneally daily for days -1 to +5); Fig. 11D shows a graph of temperatures from mice in the second experiment treated with meropenem in combination with rhu-pGSN (same regimen as in Fig. 11B). Animal temperatures were monitored every 8 hours post-infection until the end of study. Mice were euthanized upon reaching endpoint criteria (open circles) or at the study conclusion on Day 7 (closed circles).

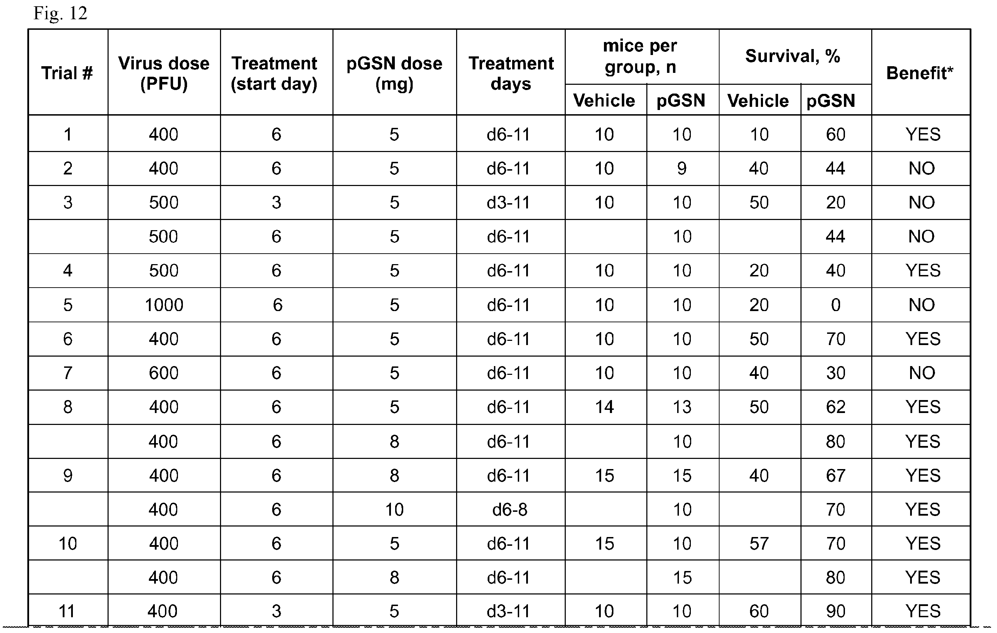

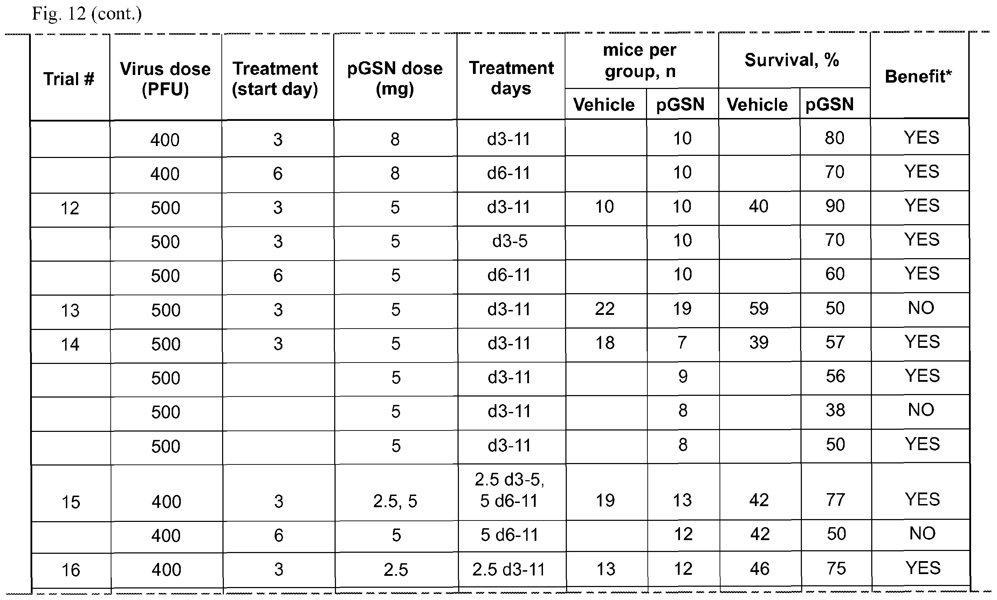

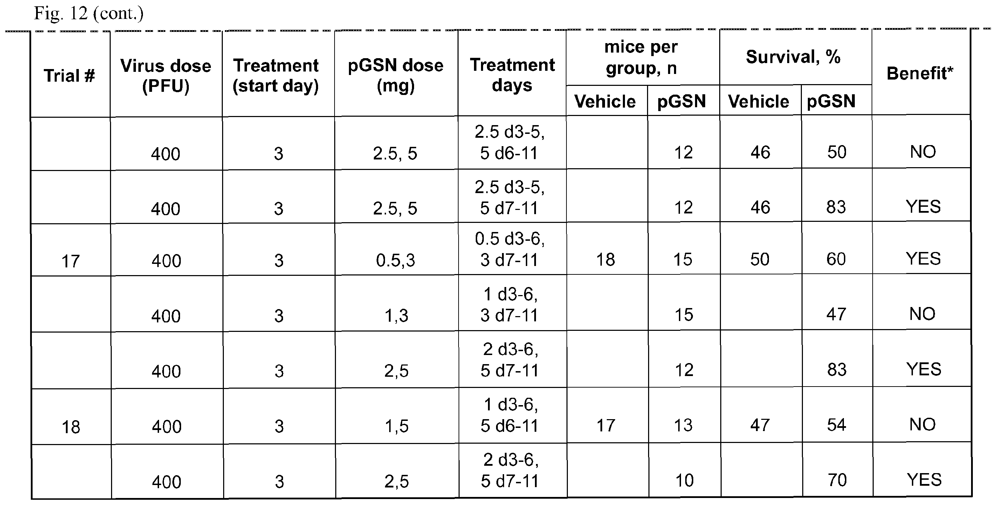

Figure 12 provides a table showing details of treatment trials using recombinant human plasma gelsolin (rhu-pGSN) in murine influenza. * = Treatment benefit scored as Yes if % survival ≥10% better with pGSN vs. Vehicle; No if % survival <10% better with pGSN.

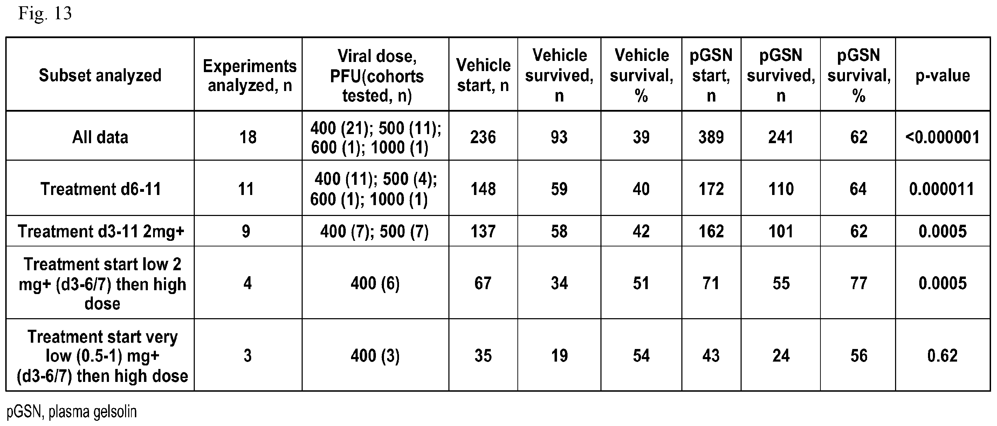

Figure 13 provides a summary of survival data using different treatment regimens. pGSN is plasma gelsolin.

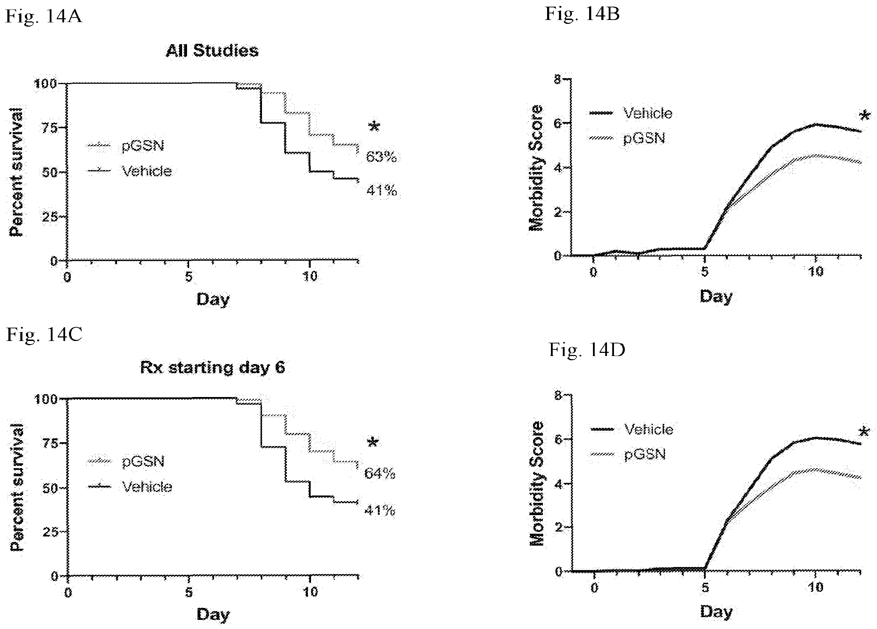

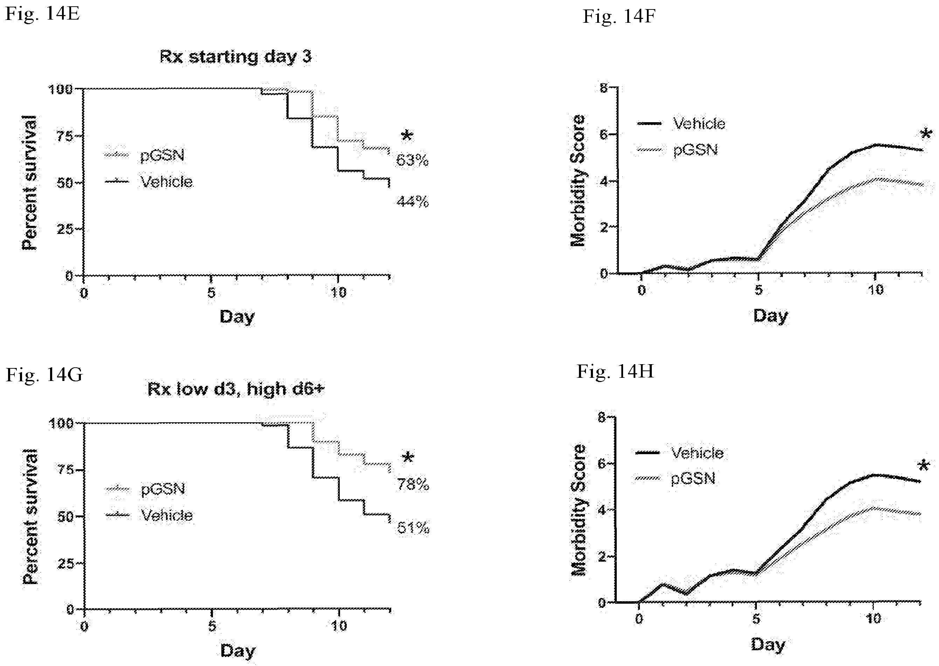

Figure 14A-H provides results of survival and morbidity analysis of different treatment regimens. Comparison of survival rates (Fig. 14A, C, E, & G) and morbidity (Fig. 14B, D, F, & H) in mice treated with rhu-pGSN or vehicle. (Fig. 14A-B) Results for all 18 trials (typically 10 or more mice per group, see details in Figs. 12 and 13) using delayed treatment. Some trials initiated treatment in different arms on day 6 or day 3. (Fig. 14C-D) Results for 13 trials using delayed treatment starting on day 6 or later. (Fig. 14E-F) Results for eight trials using treatment starting on day 3. (Fig. 14G-H) Results for four trials starting with an initially lower dose on day 3 with an increased dose starting on day 6/7. * = 0.000001, 0.00001, 0.0005, 0.0005 for Fig. 14A, C, E, & G, respectively; p < 0.0001 for Fig. 14B, D, F, & H.

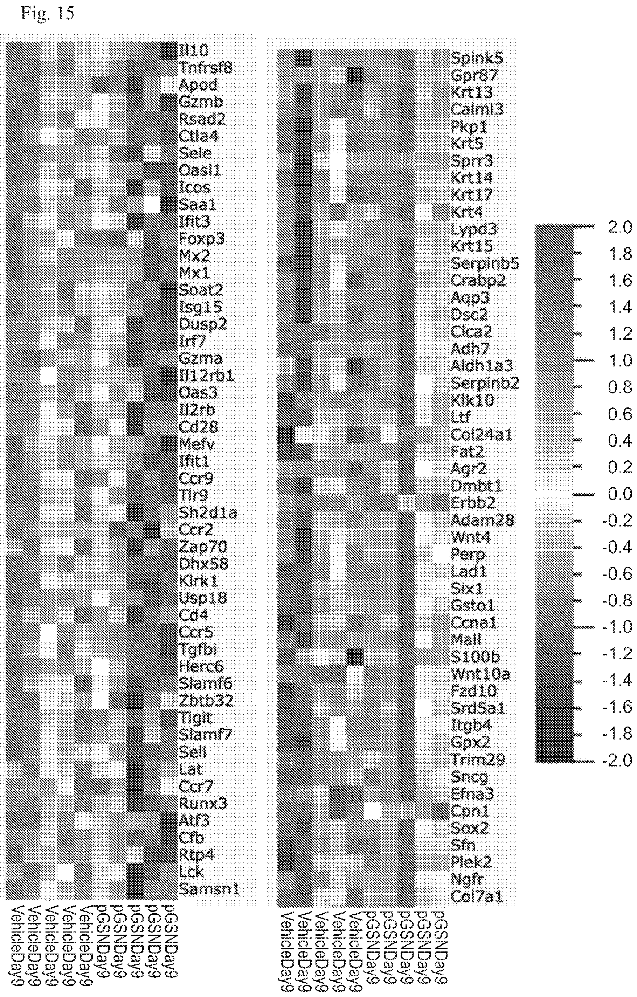

Figure 15 provides experimental results showing the top 50 up- and down- regulated differentially expressed genes in lung tissue from vehicle or rhu-pGSN treated animals (Day 9). Heat map showing top 50 down-regulated (left) and up-regulated (right) genes in the lungs of rhu-pGSN treated animals on day 9 (range -2 to + 2, shown in scale on right).

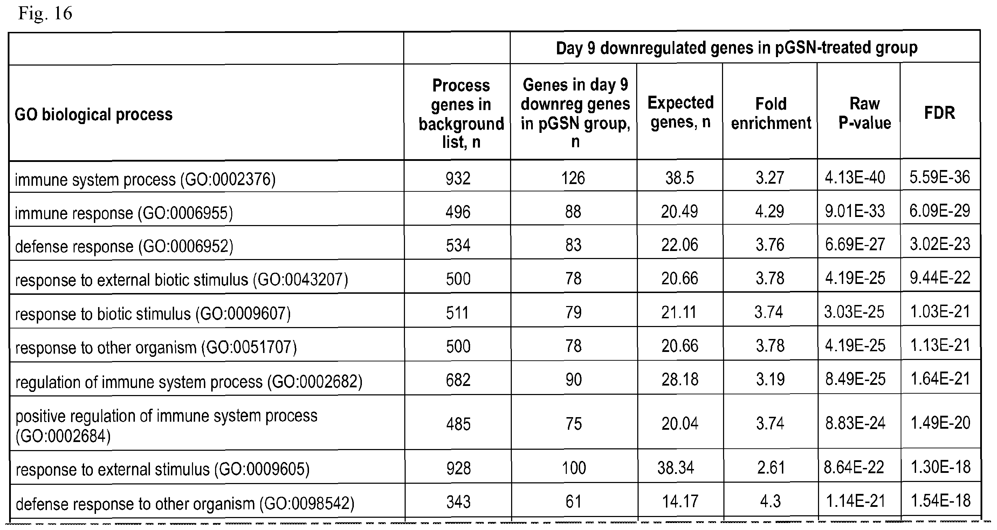

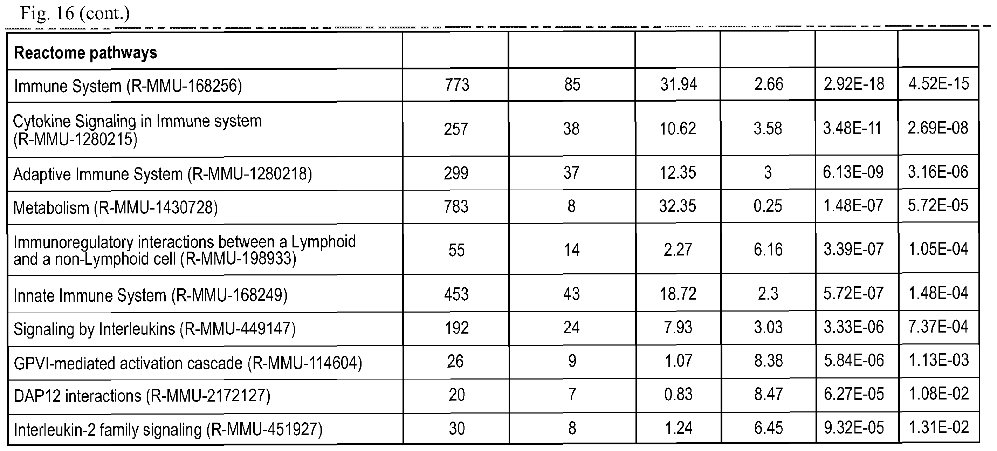

Figure 16 shows top 10 down-regulated Gene Ontology (GO) processes and pathways in plasma gelsolin (pGSN)-treated lung tissue (Day 9).

### Detailed Description

The present invention is based, in part, on the discovery that administering a gelsolin molecule and an antibacterial agent, as defined above, to a subject with a bacterial infection can result in a synergistic therapeutic effect of the two agents that reduces the bacterial infection. The invention includes a therapeutic composition comprising an exogenous gelsolin molecule and an antibacterial agent that when administered to a subject with a bacterial infection act synergistically in the subject and their synergistic action results in a therapeutic effect that is greater than the therapeutic effect of administering to the subject a clinically acceptable dose of either the gelsolin molecule or the antibacterial agent, in the absence of administering the other to the subject.

### Individual and synergistic therapeutic effects

In the claimed invention, a lack of an individual therapeutic effect of an antibacterial agent against a bacterial infection is due to antibacterial resistance in the bacteria that cause the bacterial infection. The term "resistant" used herein in relation to a bacteria or a bacterial infection means a bacteria that is not killed or reduced, respectively, by the antibacterial agent. In some embodiments of the invention, an individual therapeutic effect of an antibacterial agent on an antibacterial resistant bacteria or infection may be zero.

In the claimed invention, a bacterial infection in a subject results from a bacteria that is resistant to an individual therapeutic effect of an antibacterial agent. Acquired antibacterial resistance may be understood as an ability of a disease-causing bacteria to survive exposure to an antibacterial agent that was previously an effective treatment of the disease. A bacteria that is "antibacterial resistant" may be the cause of a bacterial infection in a subject and one or more antibacterials are ineffective against the bacterial infection, including one or more that were previously known to be therapeutically effective against the bacterial infection. In the claimed invention, a pneumococcal infection in a subject may result from the presence in the subject of a *Streptococcus pneumonia* bacterium that is resistant to a therapeutic effect of one or more β-lactam antibiotics.

It will be understood that in certain embodiments of the invention the amount of a gelsolin molecule administered and the amount of an antibacterial agent administered are each clinically acceptable amounts for administration to the subject. It is known that certain bacterial infections are not reduced by administration of an antibacterial infection administered in a clinically acceptable amount. In the invention, the bacteria that causes the bacterial infection is resistant to the administered antibacterial agent..

In some embodiments of the invention, a clinically acceptable amount of the antibacterial agent is an amount below a maximum tolerated dose (MTD) of the antibacterial agent. It is understood in the art how a MTD can be determined for an individual in order to prevent or reduce negative side effects of administering pharmacological agents. In some embodiments of the invention, an MTD of an antibacterial agent is a highest possible dose level of the antibacterial agent for the subject that is a dose that is tolerable to the subject. A tolerable dose may be determined based on side effects at a given dose level, including but not limited to: subject discomfort, physiological distress, increased risk of subject death, etc. In some embodiments of the invention, an MTD of an antibacterial agent administered to a subject is determined at least in part based on a pre-selected clinically limiting toxicity for the antibacterial agent. For example, a dose or amount of an antibacterial that is effective to reduce or kill a bacteria resistant to that antibacterial agent may when administered to a subject, result in clinically unacceptable toxicity and/or detrimental side effects in the subject.

The invention is advantageous in that it can use lower doses of antibacterial agents because of the synergistic therapeutic effects of administering both the antibacterial agent and gelsolin molecule to a subject with a bacterial infection. In some embodiments of methods of the invention synergistically effective amounts of the gelsolin molecule and the antibacterial agent decreases a minimum effective dose (MED) of the antibacterial agent in the subject. It should be understood that an amount or dose of a gelsolin molecule and an amount or dose of an antibacterial agent are independently selected, clinically acceptable amounts and doses.

In the invention, an amount of an antibacterial agent does not have an individual therapeutic effect against a bacterial infection in a subject. In the invention, an amount of a gelsolin molecule has an individual therapeutic effect against a bacterial infection that is greater than zero. Table 1 illustrates relationships between independent therapeutic effects resulting from an amount of a gelsolin molecule administered to a subject with a bacterial infection, independent therapeutic effects resulting from an amount of an antibacterial agent administered to a subject with a bacterial infection, and synergistic therapeutic effects resulting from the amount of the gelsolin molecule and the amount of the antibacterial agent administered to a subject with the bacterial infection. In each situation shown, the synergistic therapeutic effect is greater than the sum of the independent therapeutic effect of each of the gelsolin molecule and the antibacterial agent.

**Table 1. Independent and synergistic therapeutic effects of selected amount of Gelsolin molecule and selected amount of antibacterial agent.**

| Gelsolin molecule Independent Therapeutic Effect | Antibacterial Agent Independent Therapeutic Effect | Gelsolin molecule with Antibacterial Agent Synergistic Therapeutic Effect |
|---|---|---|
| 0 | 0 | >0 |
| X, where X>0 | 0 | >X |
| 0 | Y, where Y is >0 | >Y |
| X, where X >0 | Y, where Y >0 | >X + Y |

### Therapeutic Compositions and Methods

A synergistic therapeutic effect of the composition for use in treatment according to the invention, (also referred to herein as a "response" to a treatment according to the invention) can be determined, for example, by detecting one or more physiological effects of the treatment, such as the decrease or lack of symptoms following administration of the synergistic treatment. Additional means of monitoring and assessing a bacterial infection in a subject, determining one or more of presence, absence, level, severity, change in severity, etc. of bacterial infections in subjects in response to treatment are well known in the art and may be utilized in conjunction with some embodiments of methods set forth herein.

The invention includes administering a synergistic combination of a gelsolin molecule and an antibacterial agent to a subject with a bacterial infection, each in an amount effective to result in a synergistic therapeutic effect to reduce the bacterial infection in the subject. The gelsolin molecule and the antibacterial agent can be administered simultaneously. The gelsolin molecule and the antibacterial agent can be administered in the same or separate formulations, but are administered to be in the subject at the same time.

As used herein, the terms "treat", "treated", or "treating" when used in relation to a bacterial infection may refer to a prophylactic treatment that decreases the likelihood of a subject developing the bacterial infection, and may be used to refer to a treatment after a subject has developed a bacterial infection in order to eliminate or ameliorate the bacterial infection, prevent the bacterial infection from becoming more advanced or severe, and/or to slow the progression of the bacterial infection compared to the progression of the bacterial infection in the absence of a therapeutic method of the invention.

### Gelsolin molecules

Gelsolin is a highly conserved, multifunctional protein, initially described in the cytosol of macrophages and subsequently identified in many vertebrate cells (Piktel E. et al., Int J Mol Sci 2018; 19:E2516; Silacci P. et al., Cell Mol Life Sci 2004; 61:2614-23.) A unique property of gelsolin is that its gene expresses a splice variant coding for a distinct plasma isoform (pGSN), which is secreted into extracellular fluids and differs from its cytoplasmic counterpart (cGSN) by expressing an additional sequence of 25 amino acids. pGSN normally circulates in mammalian blood at concentrations of 200-300 µg/ml, placing it among the most abundant plasma proteins. In the invention a gelsolin molecule is a plasma gelsolin (pGSN) or a cytoplasmic GSN. A gelsolin molecule included in compositions for use according to the invention may be a recombinant gelsolin molecule.

A "gelsolin molecule" includes an exogenous gelsolin molecule. The term "exogenous" as used herein in reference to a gelsolin molecule means a gelsolin molecule administered to a subject, even if the same gelsolin molecule is naturally present in the subject, which may be referred to as an endogenous gelsolin molecule. A gelsolin molecule according to the invention may be a wild-type gelsolin molecule (GenBank accession No.: X04412).

### Microbial Infection

The claimed invention involves treating bacterial infection in a subject, wherein the bacterial infection is caused by a Pneumococcal species.

Characteristics of a bacterial infection in a subject that may be assessed in control subjects or groups include but are not limited to: likelihood of survival, death, body weight, level of the microbe in a biological sample from the subject, presence of the microbe in a biological sample from the subject, presence, absence, and/or level of malaise, body temperature, fever, coughing, lung exudate, congestion, headache, chills, body aches, rash, flushing, etc. It will be understood that different characteristics may be indicated in different bacterial infections and characteristics of a bacterial infection in a human may differ from characteristics of the same bacterial infection in another animal species. Characteristics present in different bacterial infections and characteristics that present in humans and/or animals are known in the art. Those of skill in the art are able to readily select one or more characteristics of a microbial infection for assessment in conjunction with use of the invention. The term "characteristics" as used herein in reference to a bacterial infection may refer to physiological symptoms of the bacterial infection.

As used herein the terms "infection" and "bacterial infection" refer to a disorder arising from the invasion of a host, superficially, locally, or systemically, by an infectious bacterial organism. The claimed invention involves treatment of a bacterial infection resistant to an antibacterial agent in a subject, wherein the bacterial infection is caused by a Pneumococcal species.

### Microbial Agents

The claimed invention involves treatment of a bacterial infection resistant to an antibacterial agent in a subject, wherein the bacterial infection is caused by a Pneumococcal species and wherein the antibacterial agent comprises a β-lactam antibiotic.

### Antimicrobial Agents

Phrases such as "antimicrobial agent", "antibacterial agent", "antiviral agent," "antifungal agent," and "anti-parasitic agent," have well-established meanings to those of ordinary skill in the art and are defined in standard medical texts. Briefly, anti-bacterial agents kill or inhibit the growth or function of bacteria. Anti-bacterial agents include antibiotics as well as other synthetic or natural compounds having similar functions. Antibiotics, typically, are low molecular weight molecules which are produced as secondary metabolites by cells, such as microorganisms. In general, antibiotics interfere with one or more bacterial functions or structures which are specific for the microorganism and which are not present in host cells.

A large class of anti-bacterial agents is antibiotics. Antibiotics that are effective for killing or inhibiting a wide range of bacteria are referred to as broad spectrum antibiotics. Other types of antibiotics are predominantly effective against the bacteria of the class grampositive or gram-negative. These types of antibiotics are referred to as narrow spectrum antibiotics. Other antibiotics which are effective against a single organism or disease and not against other types of bacteria, are referred to as limited spectrum antibiotics. Anti-bacterial agents are sometimes classified based on their primary mode of action. In general, antibacterial agents are cell wall synthesis inhibitors, cell membrane inhibitors, protein synthesis inhibitors, nucleic acid synthesis or functional inhibitors, and competitive inhibitors.

Anti-bacterial agents include but are not limited to aminoglycosides, β-lactam agents, cephalosporins, macrolides, penicillins, quinolones, sulfonamides, and tetracyclines. Examples of anti-bacterial agents include but are not limited to: Acedapsone, Acetosulfone Sodium, Alamecin, Alexidine, Amdinocillin Clavulanate Potassium, Amdinocillin, Amdinocillin Pivoxil, Amicycline, Amifloxacin, Amifloxacin Mesylate, Amikacin, Amikacin Sulfate, Aminosalicylic acid, Aminosalicylate sodium, Amoxicillin, Amphomycin, Ampicillin, Ampicillin Sodium, Apalcillin Sodium, Apramycin, Aspartocin, Astromicin Sulfate, Avilamycin, Avoparcin, Azithromycin, Azlocillin, Azlocillin Sodium, Bacampicillin Hydrochloride, Bacitracin, Bacitracin Methylene Disalicylate, Bacitracin Zinc, Bambermycins, Benzoylpas Calcium, Berythromycin, Betamicin Sulfate, Biapenem, Biniramycin, Biphenamine Hydrochloride, Bispyrithione Magsulfex, Butikacin, Butirosin Sulfate, Capreomycin Sulfate, Carbadox, Carbenicillin Disodium, Carbenicillin Indanyl Sodium, Carbenicillin Phenyl Sodium, Carbenicillin Potassium, Carumonam Sodium, Cefaclor, Cefadroxil, Cefamandole, Cefamandole Nafate, Cefamandole Sodium, Cefaparole, Cefatrizine, Cefazaflur Sodium, Cefazolin, Cefazolin Sodium, Cefbuperazone, Cefdinir, Cefditoren Pivoxil, Cefepime, Cefepime Hydrochloride, Cefetecol, Cefixime, Cefinenoxime Hydrochloride, Cefinetazole, Cefinetazole Sodium, Cefonicid Monosodium, Cefonicid Sodium, Cefoperazone Sodium, Ceforanide, Cefotaxime, Cefotaxime Sodium, Cefotetan, Cefotetan Disodium, Cefotiam Hydrochloride, Cefoxitin, Cefoxitin Sodium, Cefpimizole, Cefpimizole Sodium, Cefpiramide, Cefpiramide Sodium, Cefpirome Sulfate, Cefpodoxime Proxetil, Cefprozil, Cefroxadine, Cefsulodin Sodium, Ceftazidime, Ceftazidime Sodium, Ceftibuten, Ceftizoxime Sodium, Ceftriaxone Sodium, Cefuroxime, Cefuroxime Axetil, Cefuroxime Pivoxetil, Cefuroxime Sodium, Cephacetrile Sodium, Cephalexin, Cephalexin Hydrochloride, Cephaloglycin, Cephaloridine, Cephalothin Sodium, Cephapirin Sodium, Cephradine, Cetocycline Hydrochloride, Cetophenicol, Chloramphenicol, Chloramphenicol Palmitate, Chloramphenicol Pantothenate Complex, Chloramphenicol Sodium Succinate, Chlorhexidine Phosphanilate, Chloroxylenol, Chlortetracycline Bisulfate, Chlortetracycline Hydrochloride, Cilastatin, Cinoxacin, Ciprofloxacin, Ciprofloxacin Hydrochloride, Cirolemycin, Clarithromycin, Clavulanate Potassium, Clinafloxacin Hydrochloride, Clindamycin, Clindamycin Dextrose, Clindamycin Hydrochloride, Clindamycin Palmitate Hydrochloride, Clindamycin Phosphate, Clofazimine, Cloxacillin Benzathine, Cloxacillin Sodium, Cloxyquin, Colistimethate, Colistimethate Sodium, Colistin Sulfate, Coumermycin, Coumermycin Sodium, Cyclacillin, Cycloserine, Dalfopristin, Dapsone, Daptomycin, Demeclocycline, Demeclocycline Hydrochloride, Demecycline, Denofungin, Diaveridine, Dicloxacillin, Dicloxacillin Sodium, Dihydrostreptomycin Sulfate, Dipyrithione, Dirithromycin, Doxycycline, Doxycycline Calcium, Doxycycline Fosfatex, Doxycycline Hyclate, Doxycycline Monohydrate, Droxacin Sodium, Enoxacin, Epicillin, Epitetracycline Hydrochloride, Ertapenem, Erythromycin, Erythromycin Acistrate, Erythromycin Estolate, Erythromycin Ethylsuccinate, Erythromycin Gluceptate, Erythromycin Lactobionate, Erythromycin Propionate, Erythromycin Stearate, Ethambutol Hydrochloride, Ethionamide, Fleroxacin, Floxacillin, Fludalanine, Flumequine, Fosfomycin, Fosfomycin Tromethamine, Fumoxicillin, Furazolium Chloride, Furazolium Tartrate, Fusidate Sodium, Fusidic Acid, Gatifloxacin, Genifloxacin, Gentamicin Sulfate, Gloximonam, Gramicidin, Haloprogin, Hetacillin, Hetacillin Potassium, Hexedine, Ibafloxacin, Imipenem, Isoconazole, Isepamicin, Isoniazid, Josamycin, Kanamycin Sulfate, Kitasamycin, Levofloxacin, Levofuraltadone, Levopropylcillin Potassium, Lexithromycin, Lincomycin, Lincomycin Hydrochloride, Linezolid, Lomefloxacin, Lomefloxacin Hydrochloride, Lomefloxacin Mesylate, Loracarbef, Mafenide, Meclocycline, Meclocycline Sulfosalicylate, Megalomicin Potassium Phosphate, Mequidox, Meropenem, Methacycline, Methacycline Hydrochloride, Methenamine, Methenamine Hippurate, Methenamine Mandelate, Methicillin Sodium, Metioprim, Metronidazole Hydrochloride, Metronidazole Phosphate, Mezlocillin, Mezlocillin Sodium, Minocycline, Minocycline Hydrochloride, Mirincamycin Hydrochloride, Monensin, Monensin Sodium, Moxifloxacin Hydrochloride, Nafcillin Sodium, Nalidixate Sodium, Nalidixic Acid, Natamycin, Nebramycin, Neomycin Palmitate, Neomycin Sulfate, Neomycin Undecylenate, Netilmicin Sulfate, Neutramycin, Nifuradene, Nifuraldezone, Nifuratel, Nifuratrone, Nifurdazil, Nifurimide, Nifurpirinol, Nifurquinazol, Nifurthiazole, Nitrocycline, Nitrofurantoin, Nitromide, Norfloxacin, Novobiocin Sodium, Ofloxacin, Ormetoprim, Oxacillin Sodium, Oximonam, Oximonam Sodium, Oxolinic Acid, Oxytetracycline, Oxytetracycline Calcium, Oxytetracycline Hydrochloride, Paldimycin, Parachlorophenol, Paulomycin, Pefloxacin, Pefloxacin Mesylate, Penamecillin, Penicillin G Benzathine, Penicillin G Potassium, Penicillin G Procaine, Penicillin G Sodium, Penicillin V, Penicillin V Benzathine, Penicillin V Hydrabamine, Penicillin V Potassium, Pentizidone Sodium, Phenyl Aminosalicylate, Piperacillin, Piperacillin Sodium, Pirbenicillin Sodium, Piridicillin Sodium, Pirlimycin Hydrochloride, Pivampicillin Hydrochloride, Pivampicillin Pamoate, Pivampicillin Probenate, Polymyxin B Sulfate, Porfiromycin, Propikacin, Pyrazinamide, Pyrithione Zinc, Quindecamine Acetate, Quinupristin, Racephenicol, Ramoplanin, Ranimycin, Relomycin, Repromicin, Rifabutin, Rifametane, Rifamexil, Rifamide, Rifampin, Rifapentine, Rifaximin, Rolitetracycline, Rolitetracycline Nitrate, Rosaramicin, Rosaramicin Butyrate, Rosaramicin Propionate, Rosaramicin Sodium Phosphate, Rosaramicin Stearate, Rosoxacin, Roxarsone, Roxithromycin, Sancycline, Sanfetrinem Sodium, Sarmoxicillin, Sarpicillin, Scopafungin, Sisomicin, Sisomicin Sulfate, Sparfloxacin, Spectinomycin Hydrochloride, Spiramycin, Stallimycin Hydrochloride, Steffimycin, Sterile Ticarcillin Disodium, Streptomycin Sulfate, Streptonicozid, Sulbactam Sodium, Sulfabenz, Sulfabenzamide, Sulfacetamide, Sulfacetamide Sodium, Sulfacytine, Sulfadiazine, Sulfadiazine Sodium, Sulfadoxine, Sulfalene, Sulfamerazine, Sulfameter, Sulfamethazine, Sulfamethizole, Sulfamethoxazole, Sulfamonomethoxine, Sulfamoxole, Sulfanilate Zinc, Sulfanitran, Sulfasalazine, Sulfasomizole, Sulfathiazole, Sulfazamet, Sulfisoxazole, Sulfisoxazole Acetyl, Sulfisoxazole Diolamine, Sulfomyxin, Sulopenem, Sultamicillin, Suncillin Sodium, Talampicillin Hydrochloride, Tazobactam, Teicoplanin, Temafloxacin Hydrochloride, Temocillin, Tetracycline, Tetracycline Hydrochloride, Tetracycline Phosphate Complex, Tetroxoprim, Thiamphenicol, Thiphencillin Potassium, Ticarcillin Cresyl Sodium, Ticarcillin Disodium, Ticarcillin Monosodium, Ticlatone, Tiodonium Chloride, Tobramycin, Tobramycin Sulfate, Tosufloxacin, Trimethoprim, Trimethoprim Sulfate, Trisulfapyrimidines, Troleandomycin, Trospectomycin Sulfate, Trovafloxacin, Tyrothricin, Vancomycin, Vancomycin Hydrochloride, Virginiamycin, Zorbamycin.

The claimed invention requires an antibacterial agent that comprises a β-lactam antibiotic.

### Subjects

As used herein, a subject may be a vertebrate animal including but not limited to a human, mouse, rat, guinea pig, rabbit, cow, dog, cat, horse, goat, and primate, e.g., monkey. In certain aspects of the invention, a subject may be a domesticated animal, a wild animal, or an agricultural animal. Thus, the invention can be used to treat bacterial infections in human and non-human subjects. For instance, the invention can be used in veterinary applications as well as in human treatment regimens. In some embodiments of the invention, a subject is a human. In the invention, a subject has a bacterial infection and is in need of treatment.

The term "infected" means the day the subject is infected with the bacterial infective agent. It will be understood that the day of a subject's known or potential exposure to a bacterial agent may be regarded as day zero for the subject's infection with the bacterial agent. Exposure to a bacterial infection is understood to mean direct or indirect contact with an infected individual. A contact with an infected individual may be physical contact, contact with breath, saliva, fluid droplets, exudate, bodily fluid, discharge of an infected subject. In some embodiments, an indirect contact may be a physical contact by a subject with a substrate contaminated by the infected individual. Examples of substrates that may be contaminated by an infected individual include but are not limited to: food items, cloth, paper, metal, plastic, cardboard, fluids, air systems, etc. These and other means of exposure to bacterial infections are known in the art.

### Assessments and Controls

A bacterial infection in a subject can be detected using art-known methods, including but not limited to: assessing one or more characteristics of the bacterial infection such as, but not limited to: presence of the bacteria in a biological sample obtained from the subject; a level or amount of the bacteria in a biological sample obtained from the subject; and presence and/or level of one or more physiological symptoms of the bacterial infection detected in the subject. Characteristics of a bacterial infection detected in a subject can be compared to control values of the characteristics of the bacterial infection. A control value may be a predetermined value, which can take a variety of forms. It can be a single cut-off value, such as a median or mean. It can be established based upon comparative groups, such as in groups of individuals having the bacterial infection and groups of individuals who have been administered a treatment for the bacterial infection, etc. Another example of comparative groups may be groups of subjects having one or more symptoms of or a diagnosis of the bacterial infection and groups of subjects without one or more symptoms of or a diagnosis of the bacterial infection. The predetermined value, of course, will depend upon the particular population selected. For example, a population of individuals with the bacterial infection that have been administered a gelsolin molecule and not administered an antibacterial agent, may have a one or more different characteristics of the bacterial infection than a population of individuals having the bacterial infection that have been administered the antibacterial agent and not administered the gelsolin molecule. Accordingly, the predetermined value selected may take into account the category in which an individual falls. Appropriate categories can be selected with no more than routine experimentation by those of ordinary skill in the art.

Controls can be used in methods of the invention to compare characteristics of different control groups, characteristics of a subject with those of a control group, etc. Comparisons between subjects and controls, one control with another control, etc. may be based on relative differences. For example, though not intended to be limiting, a physiological symptom in a subject treated according to the invention comprising administering to the subject a gelsolin molecule and an antibacterial agent, can be compared to the physiological symptom of a control group that has been administered the gelsolin molecule and not administered the antibacterial agent. The comparison may be expressed in relative terms, for example, if elevated body temperature (indicative of fever), or a reduced body temperature, is a characteristic of a bacterial infection, a body temperature of a subject treated with a synergistic therapeutic method of the invention may be compared to a control level of body temperature. In some embodiments, a suitable control is a subject not treated with a synergistic therapeutic method of the invention. A comparison of a treated versus a control may include comparing percentage temperature differences between the treated subject and the selected control. In some instances, a body temperature of a subject treated with a method of the invention may be determined to be low relative to a selected control, with the comparison indicating a 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2%, 2.1%, 2.2%, 2.3%, 2.4%, 2.5%, 2.6%, 2.7%, 2.8%, 2.9%, 3%, 3.1%, 3.2%, 3.3%, 3.4%, 3.5%, 3.6%, 3.7%, 3.8%, 3.9%, 4%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, or 5.9% lower body temperature in the subject as compared to the body temperature level in the control.

In some certain instances, a body temperature of a subject treated with a method of the invention may be determined to be higher relative to a selected control, with the comparison indicating a 0.01%, 0.02%, 0.03%, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2%, 2.1%, 2.2%, 2.3%, 2.4%, 2.5%, 2.6%, 2.7%, 2.8%, 2.9%, 3%, 3.1%, 3.2%, 3.3%, 3.4%, 3.5%, 3.6%, 3.7%, 3.8%, 3.9%, 4%, 4.1%, 4.2%, 4.3%, 4.4%, 4.5%, 4.6%, 4.7%, 4.8%, 4.9%, 5.0%, 5.1%, 5.2%, 5.3%, 5.4%, 5.5%, 5.6%, 5.7%, 5.8%, or 5.9% higher lower body temperature in the subject as compared to the body temperature level in the control.

In another non-limiting example, a level of a bacterial infection can be determined using an assay to detect the presence, absence, and/or amount of the bacteria in a biological sample that is obtained from a subject having the bacterial infection. The results of the assay in a subject treated using a synergistic therapeutic method of the invention can be compared to a control level of the bacterial infection, for example results of the assays on a sample obtained from a control subject not having been so treated. Results of assays to assess a level of a bacterial infection in a subject treated using a method of the invention can be compared to a control to determine a percentage difference between the subject and the control levels. In some embodiments, a level of a treated subject's bacterial infection is less than 100% of a control infection level. In certain embodiments of the invention the level of the treated subject's bacterial infection is less than or equal to 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, 70%, 69%, 68%, 67%, 66%, 65%, 64%, 63%, 62%, 61%, 60%, 59%, 58%, 57%, 56%, 55%, 54%, 53%, 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%. 0.9%, 0.8%, 0.7%, 0.6%, 0.5%, 0.4%, 0.3%, 0.2%, or 0.1% of the control level of the bacterial infection.

In another non-limiting example, a level of a bacterial infection and/or increase in a therapeutic effect of an antibacterial agent using a method of the invention can be determined by comparing a likelihood of survival of a subject treated with a synergistic method or composition of the invention with a control likelihood of survival. A non-limiting example of a control likelihood of survival is the likelihood of survival in a subject with a bacterial infection not treated with a method of the invention. Non-limiting examples of parameters of likelihood of survival that can be measured include: determination of length of time (hours, days, weeks, etc.) a subject remains alive following a treatment of the invention, and whether a subject dies or survives following a treatment of the invention. It will be understood how these and other parameters relating to likelihood of survival can be compared to controls to assess and determine therapeutic effectiveness of a synergistic method or composition of the invention. A non-limiting example of a control of likelihood of survival is the number of days a subject survives after treatment with a synergistic method of the invention compared to the control number of days of survival in the absence of the administration of the synergistically effective amount of each of the antibacterial agent and the gelsolin molecule. In some embodiments of the invention a likelihood of survival of a subject treated with a synergistic method of the invention is at least 0.5%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, 500%, higher than a control likelihood of survival.

In another non-limiting example, a level of a bacterial infection and/or increase in a therapeutic effect of an antibacterial agent using a method of the invention can be determined by comparing level of lung pathology in a subject treated with a synergistic method or composition of the invention with a control level of lung pathology. A non-limiting example of a control level of lung pathology is the level of lung pathology in a subject with a bacterial infection not treated with a method of the invention. Non-limiting examples of parameters of lung pathology that can be measured include: determination of lung histopathology in a subject. In a non-limiting examples, histopathology of lung tissue (for example obtained via biopsy from a subject, etc.) can be assessed using art-known methods, for example, the lung tissue may be observed and scored in a blinded fashion by a board-certified pathologist. A scoring system can be used to compare a subject's lung tissue with a control. In a non-limiting example, a four-point, four-criteria system (inflammation; infiltrate; necrosis; and other, including hemorrhage) with a maximum score of 16 points may be used to evaluate lung pathology. Points for each criterion can be assigned based as no (0), minimal (1), mild (2), moderate (3), and severe (4) pathologic findings. The scoring system permits comparison of subject tissue with control tissue to assess lung pathology. Additional means of comparing lung pathology are known in the art and may be used in conjunction with methods of the invention. In some embodiments of the invention a level of lung pathology of a subject treated with a synergistic method of the invention is at least 0.5%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, 500%, lower than a control level of lung pathology.

In another non-limiting example, bacterial infection and/or increase in a therapeutic effect of an antibacterial agent using a method of the invention can be determined by comparing level of a level of a weight loss or relative weight loss in a subject treated with a synergistic method or composition of the invention with a control level of weight loss or relative weight loss. A non-limiting example of a control level of weight loss is a level of weight loss in a subject with a bacterial infection not treated with a method of the invention. Non-limiting examples of parameters of weight loss and/or relative weight loss that can be measured include: a subject's weight prior to a bacterial infection, a subject's weight during a bacterial infection prior to treatment with a synergistic method of the invention, a subject's weight after receiving a synergistic therapeutic method of the invention, etc. The subject's weight can be compared to the subject's pretreatment weight, pre-infection weight, and/or another control weight. A reduction in weight loss in the subject following the administration of the synergistic treatment of the invention, indicates a reduction in the bacterial infection in the subject. In some embodiments of the invention a level of weight loss in a subject treated with a synergistic method of the invention is at least 0.5%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, 500%, lower than a control level of weight loss.

It will be understood that controls may be, in addition to predetermined values, samples of materials tested in parallel with the experimental materials. Examples include samples from control populations or control samples generated through manufacture to be tested in parallel with the experimental samples; and also a control may be a sample from a subject prior to, during, or after a treatment with an embodiment of a method or composition of the invention. Thus one or more characteristics determined for a subject having an infection may be used as "control" values for those characteristics in that subject at a later time.

In some embodiments of the invention effectiveness of a synergistic method of the invention can be assessed by comparing synergistic therapeutic results in a subject treated using a method of the invention to one or both of: (1) an individual therapeutic effect of the gelsolin molecule and (2) an individual therapeutic effect of the antibacterial agent. In certain aspects of the invention, a difference in a level of therapeutic effectiveness may be assessed on a scale indicating an increase from a control level. In some aspects an increase is from a control level of zero obtained in (1) or (2) to a level greater than zero resulting from treatment with a synergistic method of the invention. In some embodiments of the invention, a level of therapeutic effect of a synergistic therapeutic method of the invention is an increase by at least 0.5%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, 500%, or more from a control level of therapeutic effect.

### Preparation and administration of pharmacological agents

The invention has important implications for patient treatment and also for the clinical development of new therapies. It is also expected that clinical investigators now will use the present methods for determining entry criteria for human subjects in clinical trials. Health care practitioners select therapeutic regimens for treatment based upon the expected net benefit to the subject. The net benefit is derived from the risk to benefit ratio.

The amount of a treatment may be varied for example by increasing or decreasing the amount of gelsolin molecule and/or antibacterial agent administered to a subject, by changing the therapeutic composition administered, by changing the route of administration, by changing the dosage timing and so on. The effective amount will vary with the particular infection or condition being treated, the age and physical condition of the subject being treated, the severity of the infection or condition, the duration of the treatment, the specific route of administration, and like factors are within the knowledge and expertise of the health practitioner. For example, an effective amount can depend upon the degree to which an individual has been exposed to or affected by exposure to the bacterial infection.

### Effective amounts

The term "effective amount" as used herein in relation to a treatment method or composition of the invention, is referred to as a "synergistically effect amount". Methods of the invention comprise administering each of a gelsolin molecule and an antibacterial agent in amounts that are synergistically effective amounts of the gelsolin molecule and the antibacterial agent. When administered to a subject in a method of the invention, synergistically effective amounts of the gelsolin molecule and the antibacterial agent result in a synergistic therapeutic effect against and/or a reduction in the bacterial infection in the subject.

An effective amount is a dosage of each of the pharmacological agents sufficient to provide a medically desirable result. Examples of pharmacological agents that may be used in certain embodiments of compositions and methods of the invention include, but are not limited to: gelsolin molecules and antibacterial agents. It should be understood that pharmacological agents of the invention are used to treat or prevent infections, that is, they may be used prophylactically in subjects at risk of developing an infection. Thus, an effective amount is that amount which can lower the risk of, slow or perhaps prevent altogether the development of an infection. It will be recognized when the pharmacologic agent is used in acute circumstances, it is used to prevent one or more medically undesirable results that typically flow from such adverse events.

Factors involved in determining an effective amount are well known to those of ordinary skill in the art and can be addressed with no more than routine experimentation. It is generally preferred that a maximum dose of the pharmacological agents of the invention (alone or in combination with other therapeutic agents) be used, that is, the highest safe dose according to sound medical judgment. It will be understood by those of ordinary skill in the art however, that a patient may insist upon a lower dose or tolerable dose for medical reasons, psychological reasons or for virtually any other reasons.

The therapeutically effective amount of a pharmacological agent as used in the invention is that amount effective to treat the bacterial infection. The desired response is inhibiting the progression of the infection and/or reducing the level of the infection. This may involve only slowing the progression of the infection temporarily, although it may include halting the progression of the infection permanently. This can be monitored by routine diagnostic methods known to those of ordinary skill in the art. The desired response to treatment of the infection also can be delaying the onset or even preventing the onset of the infection.

### Pharmaceutical agents and delivery

The pharmacological agents used in the methods of the invention are preferably sterile and contain an effective amount of gelsolin and an effective amount of an antibacterial agent for producing the desired response in a unit of weight or volume suitable for administration to a subject. Doses of pharmacological agents administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits. The dosage of a pharmacological agent may be adjusted by the individual physician or veterinarian, particularly in the event of any complication. A therapeutically effective amount typically varies from 0.01 mg/kg to 1000 mg/kg, from 0.1 mg/kg to 200 mg/kg, or from 0.2 mg/kg to 20 mg/kg, in one or more dose administrations daily, for one or more days. Gelsolin molecules and antibacterial agents may also be referred to herein as pharmacological agents.

Various modes of administration are known to those of ordinary skill in the art which effectively deliver the pharmacological agents used in the invention to a desired tissue, cell, or bodily fluid. The manner and dosage administered may be adjusted by the individual physician, healthcare practitioner, or veterinarian, particularly in the event of any complication. The absolute amount administered will depend upon a variety of factors, including the material selected for administration, whether the administration is in single or multiple doses, and individual subject parameters including age, physical condition, size, weight, and the stage of the disease or condition. These factors are well known to those of ordinary skill in the art and can be addressed with no more than routine experimentation.

Pharmaceutically acceptable carriers include diluents, fillers, salts, buffers, stabilizers, solubilizers and other materials that are well-known in the art. Exemplary pharmaceutically acceptable carriers are described in U.S. Pat. No. 5,211,657 and others are known by those skilled in the art. In certain embodiments of the invention, such preparations may contain salt, buffering agents, preservatives, compatible carriers, aqueous solutions, water, etc. When used in medicine, the salts may be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

Various modes of administration known to the skilled artisan can be used to effectively deliver a pharmaceutical composition as used in the invention that comprises an antibacterial agent and a gelsolin molecule to a subject to produce a synergistic therapeutic effect against a bacterial infection in the subject. Methods for administering such a composition may be topical, intravenous, oral, intracavity, intrathecal, intrasynovial, buccal, sublingual, intranasal, transdermal, intravitreal, subcutaneous, intramuscular and intradermal administration. In some embodiments of the invention, a means for administering is inhalation. The invention is not limited by the particular modes of administration disclosed herein. Standard references in the art (e.g., Remington, The Science and Practice of Pharmacy, 2012, Editor: Allen, Loyd V., Jr, 22nd Edition) provide modes of administration and formulations for delivery of various pharmaceutical preparations and formulations in pharmaceutical carriers. Other protocols which are useful for the administration of a therapeutic compound will be known to a skilled artisan, in which the dose amount, schedule of administration, sites of administration, mode of administration (e.g., intra-organ) and the like vary from those presented herein. Other protocols which are useful for the administration of pharmacological agents used in the invention will be known to one of ordinary skill in the art, in which the dose amount, schedule of administration, sites of administration, mode of administration and the like vary from those presented herein.

Administration of pharmacological agents used in the invention to mammals other than humans, e.g. for testing purposes or veterinary therapeutic purposes, is carried out under substantially the same conditions as described above. It will be understood by one of ordinary skill in the art that this invention is applicable to both human and animal diseases. Thus, this invention is intended to be used in husbandry and veterinary medicine as well as in human therapeutics. A pharmacological agent may be administered to a subject in a pharmaceutical preparation.

When administered, the pharmaceutical preparations are applied in pharmaceutically-acceptable amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts.

A pharmacological agent or composition may be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application. The components of the pharmaceutical compositions also are capable of being co-mingled with the pharmacological agents used in the invention, and with each other, in a manner such that there is no interaction which would substantially impair the desired pharmaceutical efficacy.

The pharmaceutical compositions may contain suitable buffering agents, as described above, including: acetate, phosphate, citrate, glycine, borate, carbonate, bicarbonate, hydroxide (and other bases) and pharmaceutically acceptable salts of the foregoing compounds. The pharmaceutical compositions also may contain, optionally, suitable preservatives, such as: benzalkonium chloride; chlorobutanol; parabens and thimerosal.

The pharmaceutical compositions may conveniently be presented in unit dosage form and may be prepared by any of the methods well known in the art of pharmacy. All methods include the step of bringing the active agent into association with a carrier, which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, pills, lozenges, each containing a predetermined amount of the active compound (e.g., gelsolin). Other compositions include suspensions in aqueous liquids or nonaqueous liquids such as a syrup, elixir, an emulsion, or a gel.

Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers, i.e. EDTA for neutralizing internal acid conditions or may be administered without any carriers.

Also specifically contemplated are oral dosage forms of the above component or components. The component or components may be chemically modified so that oral delivery of the derivative is efficacious. Generally, the chemical modification contemplated is the attachment of at least one moiety to the component molecule itself, where said moiety permits (a) inhibition of proteolysis; and (b) uptake into the blood stream from the stomach or intestine. Also desired is the increase in overall stability of the component or components and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline. Abuchowski and Davis, 1981, "Soluble Polymer-Enzyme Adducts" In: Enzymes as Drugs, Hocenberg and Roberts, eds., Wiley-Interscience, New York, N.Y., pp. 367-383; Newmark, et al., 1982, J. Appl. Biochem. 4:185-189. Other polymers that could be used are poly-1,3-dioxolane and poly-1,3,6-tioxocane.

For the pharmacological agent the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of gelsolin molecule and/or the antibacterial agent or by release of the biologically active material beyond the stomach environment, such as in the intestine.

Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

For administration by inhalation, the compounds for use according to the present invention may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Also contemplated herein is pulmonary delivery of gelsolin. Gelsolin is delivered to the lungs of a mammal while inhaling and traverses across the lung epithelial lining to the blood stream.

Nasal (or intranasal) delivery of a pharmaceutical composition as used in the present invention is also contemplated. Nasal delivery allows the passage of a pharmaceutical composition as used in the present invention to the blood stream directly after administering the therapeutic product to the nose, without the necessity for deposition of the product in the lung. Formulations for nasal delivery include those with dextran or cyclodextran.

The compounds, when it is desirable to deliver them systemically, may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions. Alternatively, the active compounds may be in powder form for constitution with a suitable vehicle, e.g., sterile pyrogen-free water, before use.

Pharmacological agent(s), including specifically but not limited to a gelsolin molecule and an antibacterial agent, may be provided in particles. Particles as used herein means nano or microparticles (or in some instances larger) which can consist in whole or in part of gelsolin or the antibacterial agent as described herein. The particles may contain the pharmacological agent(s) in a core surrounded by a coating, including, but not limited to, an enteric coating. The pharmacological agent(s) also may be dispersed throughout the particles. The pharmacological agent(s) also may be adsorbed into the particles. The particles may be of any order release kinetics, including zero order release, first order release, second order release, delayed release, sustained release, immediate release, and any combination thereof, etc. The particle may include, in addition to the pharmacological agent(s), any of those materials routinely used in the art of pharmacy and medicine, including, but not limited to, erodible, nonerodible, biodegradable, or nonbiodegradable material or combinations thereof. The particles may be microcapsules which contain the gelsolin in a solution or in a semi-solid state. The particles may be of virtually any shape.

Both non-biodegradable and biodegradable polymeric materials can be used in the manufacture of particles for delivering the pharmacological agent(s). Such polymers may be natural or synthetic polymers. The polymer is selected based on the period of time over which release is desired. Bioadhesive polymers of particular interest include bioerodible hydrogels described by H. S. Sawhney, C. P. Pathak and J. A. Hubell in Macromolecules, (1993) 26:581-587. These include polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

The pharmacological agent(s) may be contained in controlled release systems. The term "controlled release" is intended to refer to any drug-containing formulation in which the manner and profile of drug release from the formulation are controlled. This refers to immediate as well as non-immediate release formulations, with non-immediate release formulations including but not limited to sustained release and delayed release formulations. The term "sustained release" (also referred to as "extended release") is used in its conventional sense to refer to a drug formulation that provides for gradual release of a drug over an extended period of time, and that preferably, although not necessarily, results in substantially constant blood levels of a drug over an extended time period. The term "delayed release" is used in its conventional sense to refer to a drug formulation in which there is a time delay between administration of the formulation and the release of the drug therefrom. "Delayed release" may or may not involve gradual release of drug over an extended period of time, and thus may or may not be "sustained release."

Use of a long-term sustained release implant may be particularly suitable for treatment of chronic conditions. "Long-term" release, as used herein, means that the implant is constructed and arranged to deliver therapeutic levels of the pharmacological agent(s) for at least 7 days, and preferably 30-60 days. Long-term sustained release implants are well-known to those of ordinary skill in the art and include some of the release systems described above.

The disclosure also includes kits. In some aspects of the disclosure, the kit can include one or more pharmaceutical preparation vial, a pharmaceutical preparation diluent vial, an antibacterial agent and a gelsolin molecule. A vial containing the diluent for the pharmaceutical preparation is optional. A diluent vial may contain a diluent such as physiological saline for diluting what could be a concentrated solution or lyophilized powder of the gelsolin molecule and/or the antibacterial agent. The instructions can include instructions for mixing a particular amount of the diluent with a particular amount of the concentrated pharmaceutical preparation, whereby a final formulation for injection or infusion is prepared. The instructions may include instructions for treating a subject with effective amounts of the gelsolin molecule and the antibacterial agent. It also will be understood that the containers containing the preparations, whether the container is a bottle, a vial with a septum, an ampoule with a septum, an infusion bag, and the like, can contain indicia such as conventional markings that change color when the preparation has been autoclaved or otherwise sterilized.

The present invention is further illustrated by the following Examples, which in no way should be construed as further limiting.

The following examples are provided to illustrate specific instances of the practice of the present invention and are not intended to limit the scope of the invention. As will be apparent to one of ordinary skill in the art, the present invention will find application in a variety of compositions for use in treatment.

### Examples

### Example 1 (comparative)

Antibiotic resistant pneumococcal pneumonia occur and can be problematic. Studies have been conducted to assess a novel therapeutic strategy for combating infections that includes means to augment innate immunity. Experiments were performed to determine the effect of pGSN administration on macrophages and host survival.

### Methods

### Bacterial strains and culture

*S. pneumoniae* serotype 3 (catalog no. 6303, American Type Culture Collection, Rockville, MD) were cultured overnight on 5% sheep blood-supplemented agar petri dishes (catalog no. 90001-282, VWR, West Chester, PA) and prepared and quantified as previously reported (Yang Z. et al., Am J Physiol Lung Cell Mol Physiol 2015; 309:L11-6).

### In vitro and in vivo procedures

### (1) In vitro studies

*In vitro* studies were performed in which 125-250 µg/ml pGSN was added to bacterial cultures and bacterial survival was determined.

### (2) In vivo studies

B16 mice were challenged with 10⁵ pneumococci by i.n. insufflation and were administered 10 mg pGSN s.c. 2 h before and 8 and 20 h after the infection. In some studies the pGSN was administered as an aerosol for 15 or 30 minutes prior to infection. The aerosol was generated as in Hamada, K., et al, J. Immunology. 2003;170(4):1683-9, using a solution of 5 mg/ml.

### Results/Discussion

Results of *in vitro* studies demonstrated that pGSN improved macrophage uptake (Fig. 1A) and killing of internalized pneumococci (Fig. 1B) when present at 125-250 µg/ml, which is similar to normal plasma levels. *In vivo,* pGSN (10 mg s.c. 2 h before and 8 and 20 h after infection improved bacterial clearance (fewer surviving bacteria at 24h) in Bl6 mice challenged with 10⁵ pneumococci by i.n. insufflation (Fig. 1C); similar results were seen when pGSN was administered as an aerosol for 15 or 30 minutes prior to infection; aerosol generated as in Hamada, K., et al, J. Immunology. 2003;170(4):1683-9 using a solution of 5 mg/ml (Fig. 1D). Systemic pGSN (s.c.) improves survival in primary (Fig. 1E, using 3 X 10⁵ CFU inoculum) or secondary post-influenza pneumococcal pneumonia (Fig. 1F, using 500 CFU inoculum on day 7 after mild influenza infection with PR8) even in the absence of any antibiotic treatment. * = p<.05 vs control, n= 6-12 per group. All experiments used serotype 3 Strep. Pneumoniae [ATCC #6303].

Macrophage NOS3 is an important mechanism for host defense against pneumonia in mice, and also functions in human macrophages (Yang, Z., et al., Elife. 2014;3. Epub 2014/10/16. Doi 10.7554/elife.03711). Results indicated that this pathway functions as an important mechanism for pGSN effects on macrophages, because the pGSN was unable to improve bacterial killing response in NOS3-deficient macrophages (Fig. 2A) and in NOS3 deficient mice (Fig. 2B).

Additional studies were performed using *E. Coli* and *Francisella tularensis* (see Yang. Z., et al, American Journal of Physiology Lung Cellular and Molecular Physiology. 2015;309(1):L11-6).

### Example 2

Studies were performed to evaluate effects of pGSN treatment on antibiotic-sensitive and antibiotic-resistant mouse models of pneumococcal pneumonia.

### Methods

### Bacterial strains and culture

*S. pneumoniae* serotypes 3 and 14 (Catalog nos. 6303 and 700677, respectively) were obtained from the American Type Culture Collection (Rockville, MD). Serotype 3 bacteria were cultured overnight on 5% sheep blood-supplemented agar petri dishes (Catalog no. 90001-282, VWR, West Chester, PA) and prepared and quantified as previously reported (Yang Z. et al., Am J Physiol Lung Cell Mol Physiol 2015; 309:L11-6). Because serotype 14 required a more detailed protocol to achieve consistent results, the growth protocol reported in Restrepo AV et al., BMC Microbiol 2005; 5:34 was followed, which uses two sequential expansions in liquid broth culture before centrifugation and adjustment of bacterial concentration by OD600 for *in vivo* administration.

### Mouse models of pneumococcal pneumonia

Normal 6- to 8-week (wk) old male CD1 mice were obtained from Charles River Laboratories (Wilmington, MA). Primary pneumococcal pneumonia was induced as previously reported (Yang Z. et al., Am J Physiol Lung Cell Mol Physiol 2015; 309:L11-6.) For antibiotic-sensitive pneumonia, intranasal instillation of 1.5-2 x 10⁶ colony-forming units (CFU) of *Streptococcus pneumoniae* type 3 was performed into mice under anesthesia with ketamine (72 mg/kg i.p.) plus xylazine (9.6 mg/kg i.p.). *Streptococcus pneumoniae* type 14, which is resistant to penicillin (minimum inhibitory concentration (MIC) = 8 µg/ml) and other antibiotics (Jabes D. et al., J Infect Dis 1989; 159:16-25), was used to model antibiotic-resistant pneumonia. For this pathogen, range-finding experiments identified a high lethality inoculum of approximately 300 x 10⁶ colony-forming units (CFU) which was used for instillation under anesthesia as above. Most trials used 10 mice per group for the vehicle, penicillin (PEN), pGSN or PEN + pGSN groups.

### Treatments and outcomes

Recombinant human pGSN (rhu-pGSN) was synthesized in *E. coli* and purified by Fujifilm Diosynth (Billingham, UK). rhu-pGSN was administered to mice by intraperitoneal injection at doses ranging from 5-10 mg as detailed in Results. In some experiments, penicillin (G Procaine Injectable Suspension, NDC 57319-485-05, Phoenix Pharmaceuticals) was administered by i.m. injection of 0.1-2 mg. The mice were monitored for 10 days, measuring survival, changes in weight and overall morbidity using a composite index (*i.e.,* 1 point each for hunched appearance, ruffled fur or partly closed eyes; 1.5 points for prolapsed penis or splayed hind quarter; 2 points for listlessness, with a maximum score of 8; the assessment was performed without blinding to treatment group) adapted from guidelines in Burkholder T. et al., Curr Protoc Mouse Biol 2012; 2:145-65. Weights and morbidity scores for the last day alive were carried forward for animals that did not survive. To assess lung inflammation by quantifying neutrophil influx, one cohort of animals underwent lung lavage at 48 hours following infection after euthanasia as previously described (Yang Z. et al., Am J Physiol Lung Cell Mol Physiol 2015; 309:L11-6; and Yang Z. et al., Elife 2014; 3. After centrifugation, resuspended lavage samples were counted by hemocytometer and differential cell counts were performed on Wright-Giemsa stained cytocentrifuge preparations.

### Statistical analysis

Data were analyzed using Prism (GraphPad Software) or SAS (SAS Institute) software. Differences in Kaplan-Meier survival curves were analyzed using a log-rank test with Sidak adjustment for multiple comparisons. For other measurements, differences between groups were examined by ANOVA.

### Results

Delayed treatment with rhu-pGSN was tested in the same murine model used previously to demonstrate improved survival with pre-treatment (Yang Z. et al., Am J Physiol Lung Cell Mol Physiol 2015; 309:L11-6). As shown in Fig. 3A, pGSN treatment given only on days 2 and 3 after infection with serotype 3 pneumococci led to substantially improved survival from a highly lethal inoculum compared to vehicle controls, even in the absence of antibiotic treatment. To contrast with subsequent experiments using serotype 14, the 100% survival of antibiotic-treated mice confirmed that serotype 3 was highly sensitive to penicillin (Fig. 3B).

To determine if these findings extended to an antibiotic-resistant pneumonia, a similar model was developed using highly virulent serotype 14 pneumococci. Treatments were begun at 24 hours after infection and continued daily for 9 days. Mice treated with only the diluent vehicle experienced high mortality (Fig. 4A). Penicillin treatment alone had no benefit (Fig. 4A), consistent with the reported in vitro high-level resistance of this bacterial strain (Jabes D. et al., J Infect Dis 1989; 159:16-25).

During the 24 hours prior to treatment, all the mice experienced identical deterioration evidenced by equivalent weight loss and morbidity scores. Neutrophil influx at 48 hours after infection was decreased in animals treated with a single dose of pGSN with or without penicillin (total lavage neutrophils x 10E4 in vehicle, PEN, pGSN and PEN + pGSN groups respectively: 186 ± 54, 153 ± 74, 111 ± 16, 104 ± 20; p < .03, n = 5-6/group). rhu-pGSN treatment alone caused substantial improvement in overall survival, recovery from weight loss, and improvement in morbidity scoring (Figs. 4A-C).

*In vitro,* penicillin treatment alone or in combination with pGSN had no effect on bacterial growth (increase in bacterial CFU, 1 hour (h) culture with vehicle, PEN (16 µg/ml) or PEN + pGSN (250 µg/ml): 88000, 105000, 88000, respectively, averaging 2 replicates).

*In vivo,* treatment with a combination of penicillin and pGSN resulted in higher survival than with pGSN alone (Fig. 4A), but this was not was not statistically different when adjusted for multiple comparisons (p = 0.47, see Fig. 5). Results of all survival experiments are presented in Fig. 5 and show that for each of the nine experiments, survival was highest in the pGSN + PEN group, followed by pGSN alone compared to either PEN or vehicle alone (and the pGSN + PEN combination was significantly better than pGSN alone). The Fig. 5 table provides results from nine experiments in which testing delayed administration of four treatments was assessed. Data from the final four experiments, which used essentially identical treatments and were representative of the overall results obtained in all nine studies, are shown in Fig. 4A-C. Fig. 5 provides details of all nine experiments, including pilot and range-finding trials. Column H shows a change in bacterial growth method obtained using a method for 2X growth in BHI broth for penicillin-resistant pneumococci (Restrepo AV et al., BMC Microbiol 2005; 5:34) for superior growth results. The survival differences were statistically significant, as determined by analysis of all the nine studies pooled using log rank analysis along with Sidak correction for multiple comparisons. Details of the results of statistical analysis of the final four experiments (#6-9) are summarized in Fig. 4A-C.

### Discussion

Studies were designed and configured to mimic the clinical situation where a subject presents after an infection is apparent. Therefore, the experiments were performed using a clinically relevant scenario of delaying administration until the mice had become visibly ill rather than the pre- or concurrent treatment used in prior studies (Yang Z. et al., Am J Physiol Lung Cell Mol Physiol 2015; 309:L11-6.). This design was used to evaluate the potential of pGSN to improve treatment outcomes. The key findings were that delayed pGSN treatment improved survival, either when used alone without an antibiotic or in combination with a suboptimal antibiotic to which the bacterial strain is highly resistant. The observed lowered bronchoalveolar neutrophil counts in infected pGSN-treated animals may reflect accelerated bacterial clearance by pGSN-stimulated resident macrophages, pGSN's inflammationmodulating activity, or both. With serotype 14, the ability to study longer delays before therapy was limited in pilot trials by the relatively high number of deaths by day 2 or 3 without treatment. Studies are performed to examine other antibiotic-resistant organisms in other model systems. Previous findings that pGSN enhances microbicidal function of macrophages against other bacteria (*e.g., E. coli, F. tularensis* LVS [Yang Z. et al., Am J Physiol Lung Cell Mol Physiol 2015; 309:L11-6.]) are encouraging in this regard, but direct testing is needed.

The totality of the data suggests a synergistic interaction of pGSN with penicillin treatment that had no effects by itself. However, this conclusion relies on pooled analysis of all the range-finding as well as final trials performed. When only the final four replicate studies (Figs. 4A-C) are analyzed, the comparison is in the same direction but does not achieve statistical significance. Potentiation of penicillin effects on bacterial growth in vitro by concomitant rhu-pGSN was not observed. While not intending to be bound by any particular theory, these data suggest that antibacterial defenses enhanced by pGSN may be even more effective against bacteria that are slightly perturbed (but not killed) by penicillin. The mechanism merits future attention, especially if similar results are observed in other infections with resistant bacteria. In summary, rhu-pGSN can improve outcomes in a highly lethal pneumococcal pneumonia model when given after a clinically relevant delay, even in the setting of antibacterial resistance. These findings support further evaluation of pGSN as an adjunctive therapy for serious antibiotic-resistant infections.

### Example 3 (comparative)

Studies were performed to evaluate effects of rhu-pGSN treatment to meropenem in highly lethal, multidrug-resistant *P. aeruginosa* pneumonia in a neutropenic mouse model.

### Methods

### Production of rhu-pGSN

Recombinant human plasma gelsolin (rhu-pGSN), was produced in *E. coli* and subsequently lyophilized for reconstitution. Vehicle controls containing formulation components were used for the comparator mice.

### Bacteria strain and growth conditions

*P. aeruginosa* UNC-D is a sputum isolate from a patient with cystic fibrosis. [Lawrenz MB, et al. Pathog. Dis. 73 (2015)]. Bacteria were cultured on trypticase soy agar (TSA) plates and in Lennox broth at 37°C with shaking of broth cultures. Minimum inhibitory concentrations of the UNC-D strain are: ceftazidime [32 µg/ml], meropenem [8 µg/ml], imipenem [16 µg/ml], tobramycin [32 µg/ml], piperacillin [16 µg/ml], aztreonam [4 µg/ml], colistin [1 µg/ml], and fosfomycin [256 µg/mL]. Bacteria were prepared for animal challenge studies by culturing bacteria in Lennox broth overnight and washing the bacteria into 1X PBS before diluting to a final concentration based on OD₆₀₀-based estimates and a final 50 µl delivery dose. Bacterial inocula were confirmed by serial dilution and colony enumeration on TSA plates.

### Animal respiratory infection model

The BALB/c infection model of *P. aeruginosa* UNC-D strain [Lawrenz MB, et al. (2015) Pathog. Dis. 73(5):ftv025] was specifically designed to test for adjunctive therapies that might result in improved efficacy of failing meropenem monotherapy against a multidrug resistant (MDR) *P. aeruginosa* UNC-D strain resistant to several clinically important antibiotics including meropenem. Previous experience demonstrated this model is most informative when examining novel compounds using meropenem doses that provide approximately 50% mortality with meropenem treatment alone [Lawrenz MB, et al. (2015) Pathog. Dis. 73(5):ftv025]. Mice were housed and treated in accordance with standard animal experimentation guidelines at the University of Louisville. Briefly, female BALB/c mice were rendered neutropenic using cyclophosphamide injections (150 mg/kg) on days -5 and -3 prior to infection, typically resulting in ~90% drop in the neutrophil counts. Approximately 10^{5.5} CFU of UNC-D was directly instilled into the lungs by intubationmediated intratracheal instillation. Meropenem (Hospira; Lake Forest, IL) was administered by subcutaneous injection beginning at 3 hours post-infection and q8h for 5 days.

To determine if rhu-pGSN adjunctive therapy improves the efficacy of meropenem, 12 mg/day of rhu-pGSN was administered by intraperitoneal injection of 0.3 ml at -24, -3, 3, 27, 51, 75, 99, and 123 hours post-infection. Mice were monitored for development of illness every 8 hours after infection for 7 days, including temperatures measured via transponders implanted subcutaneously prior to the initiation of the studies (BioMedic Data Systems; Seaford, DE). Moribund mice were humanely euthanized and scored as succumbing to the infection at the next time point. Tissues samples were harvested for bacterial counts and pathology as previously described [Lawrenz MB, et al. Pathog. Dis. 73 (2015)]. Mice surviving to 7 days were scored as surviving infection and euthanized; tissues were similarly processed. Lung histopathology was scored in a blinded fashion by a board-certified veterinary pathologist. A four-point, four-criteria system (inflammation; infiltrate; necrosis; and other, including hemorrhage) with a maximum score of 16 points was used to evaluate lung pathology. Points for each criterion were assigned based as no (0), minimal (1), mild (2), moderate (3), and severe (4) pathologic findings.

### Statistical analyses

In total, 3 comparable experiments were independently performed using this model. Titration experiments were done when a new batch of meropenem was to be used to estimate the effective dose (ED)₅₀ for each lot of antibiotic prior to the formal experiments. Overall survival and survival with minimal lung injury (defined post hoc as histopathology scores ≤ 2) were tallied for the experiments overall and for experimental conditions where the meropenem-only control groups protected ≤ 50% of the mice. The 95% confidence intervals and p-values for differences in the proportions of surviving mice between treatment arms with and without rhu-pGSN were computed via normal approximation to the binomial distribution. For the individual experimental conditions where the mortality rate in the control meropenem group approximated 50% or more, survival curves were analyzed by the log rank test, temperature data were analyzed by two-way ANOVA, and bacterial burden and pathology scores were analyzed by one-way ANOVA with Tukey posttest multiplicity adjustment. The prespecified primary endpoint was survival 7 days post-infectious challenge. During analysis of these data, a "survival-plus" endpoint to examine survival with healthy lungs (histopathology score ≤ 2) was used as a clinically meaningful extension of a good outcome. Bacterial burden and temperature response were not included in this twopronged composite because they were not direct measures of clinical improvement.

### Results

### rhu-pGSN improved survival of mice infected with P. aeruginosa

To determine whether rhu-pGSN could improve the efficacy of meropenem against pulmonary infection, female BALB/c mice were made neutropenic with cyclophosphamide (n=8), infected with MDR *P. aeruginosa,* and treated with varying doses of meropenem to determine the dose at which meropenem therapy begins to fail in this model (*i.e.,* approached the ED₅₀ for meropenem). Mice were treated with the selected doses of meropenem with or without rhu-pGSN for 5 days post-infection and monitored for the development of moribund disease for 7 days post-infection (Fig. 6). In both experiments 1 and 2, treatment with 1250 mg/kg/day of meropenem resulted in ≤ 50% survival, indicating failure of meropenem treatment and allowing ascertainment of whether adjunctive therapy with rhu-pGSN could improve efficacy. Focusing on animals receiving this dose, addition of rhu-pGSN numerically increased the number of animals that survived to the end of each study (Fig. 7A-B). Combining the two sequential studies, 31% of the mice receiving meropenem alone survived for 7 days compared with 75% survival when mice were given meropenem with rhu-pGSN (Δ (95% confidence interval) = 44% (13, 75); p = 0.0238; Fig. 7C). A third experiment using a different lot of meropenem that demonstrated a higher than predicted meropenem efficacy (75% survival in meropenem only group) did not show a difference in survival rates between the treatment groups (Fig. 6).

To ascertain if the increased survival with rhu-pGSN therapy was associated with decreased bacterial burden in the lungs, colony counts were determined from the lungs of mice receiving 1250 mg/kg/day at the time of euthanasia (Fig. 8A-C). A general trend was observed suggesting that rhu-pGSN improved control of bacterial burden in the lungs of infected mice compared to meropenem alone but a statistically significant difference in bacterial counts was only observed in the second study (p = 0.0273).

Overall survival for all the dosing groups in the 3 experiments combined was 35/64 (55%) and 46/64 (72%) in mice treated with meropenem without or with rhu-pGSN, respectively [Δ (95% confidence interval) = 17% (1, 34)]. Although treatment with adjunctive rhu-pGSN increased the efficacy of meropenem against pulmonary infection with *P. aeruginosa,* inhibition of bacterial proliferation in the lungs may only partially explain the observed benefit. Interestingly, it was observed that meropenem alone controlled spread from lung to spleen in both studies, but that pGSN allowed splenic colonization in some animals. While this observation was not significant in any study alone, combining data demonstrated a significant increase in splenic counts in pGSN-treated mice. **In** conjunction with improved survival, these observations were consistent with rhu-pGSN exerting an opsonic effect which enhanced splenic uptake.

### rhu-pGSN limits acute lung injury

The lack of an unambiguous relationship between reduced bacterial loads in the lungs and increased survival in mice that received rhu-pGSN raised the possibility that rhu-pGSN protection might be mediated by alternative or additional mechanisms. Because pGSN modulates inflammation, the question of whether adjunctive rhu-pGSN therapy diminished lung injury was investigated in *P. aeruginosa* infected animals receiving 1250 mg/kg/day. Representative sections of lung tissue harvested from animals were blindly scored for pathology by a board-certified veterinary pathologist. Addition of rhu-pGSN to meropenem reduced host lung damage (Fig. 9A-B; p = 0.0035 and p = 0.1514, respectively). Combining the data from these two independent studies, the mean pathology score for mice receiving meropenem alone was 6.86, whereas the mean pathology score for mice that received both meropenem and rhu-pGSN was 2.53 (Fig. 9C; p = 0.0049).

Based on these observations that rhu-pGSN protected against lung damage, the analysis was expanded to include mice receiving doses of meropenem above and below 1250 mg/kg/day. Overall survival of mice receiving different doses of meropenem for three individual experiments are shown in Fig. 6. Animals surviving infection for 7 days were grouped as either demonstrating near normal lung histology (pathology scores ≤ 2) or signs of lung pathology (pathology scores > 2). Retrospectively using this criterion, overall survival with minor lung injury was found in 26/64 (41%) mice receiving only meropenem versus 38/64 (59%) mice given meropenem plus rhu-pGSN [Δ (95% confidence interval) = 19% (2, 36)] (Fig. 10). To eliminate the noise generated by highly effective and ineffective meropenem doses, arbitrary but clinically reasonable exclusion limits of ≥ 75% and ≤ 25% were then imposed for the control survival rate. In this middle ground of responsiveness to meropenem alone, another exploratory post-hoc analysis yielded favorable outcomes (survival with near-normal lungs) in 12/32 (37.5%) with only meropenem and in 27/32 (84.4%) with the combination of meropenem and rhu-pGSN [Δ = 47% (26, 68)].

Using surviving mice as the denominator, near-normal lung histopathology was found in 26/35 (74.3%) and 38/46 (82.6%), respectively, with meropenem treatment alone versus meropenem and rhu-pGSN combined therapy. These data together indicate that addition of rhu-pGSN may decrease lung injury caused by *P. aeruginosa* infection treated only with antibacterial agents.

### Plasma gelsolin speeds resolution of the host systemic response

As part of monitoring disease progression, host temperature was followed over the course of infection. For this model, all mice tended to exhibit a steady decrease in body temperature within the first 24 hours of infection. For mice that received efficacious treatments, their temperatures eventually returned to normal, while the temperature of mice that received sub-efficacious treatments continued to decline [Lawrenz MB, et al. (2015) Pathog. Dis. 73(5):ftv025]. The time course of temperature normalization allowed assessment of differences in recovery rates between different treatments. Focusing on the dosing regimens approaching the targeted ED₅₀ for meropenem alone in these experiments, the question of whether pGSN sped the restoration of temperature homeostasis in mice surviving infection was investigated. In the two studies achieving a survival advantage, mice typically experienced an ~10°F decrease in body temperature within the first 24 hours after infection (Fig. 11A-D). Mice treated with meropenem alone who were to survive to Day 7 began to restore their body temperatures toward 95° F within 3-5 days post-infection. In contrast, the restoration of host body temperature was much more rapid in mice treated with rhu-pGSN and meropenem, where survivor body temperatures returned to 95°F by Day 2. Thus, adjunctive rhu-pGSN not only improved survival and lung pathology, but also accelerated systemic recovery of the host as measured by temperature curves. In the third experiment where a survival advantage with rhu-pGSN was not seen, no difference in the temperature course was observed between treatment arms.

### Discussion

rhu-pGSN improved survival when added to meropenem in an established murine model of severe multidrug-resistant *P. aeruginosa* pneumonia. Normalization of temperature in surviving mice generally occurred more rapidly with adjunctive rhu-pGSN therapy than with meropenem alone. Lungs from rhu-pGSN recipients generally had fewer viable bacteria. Furthermore, rhu-pGSN reduced the degree of acute lung injury in surviving animals, which potentially represents a clinically important advance in the treatment of serious bacterial pneumonia. Taken together, these findings suggest that survival advantage afforded by the addition of rhu-pGSN to meropenem treatment was likely due in large part a rhu-pGSN-mediated reduction in the bacterial load and severity of lung injury during the course of infection.

The first line of host defense against infection involves a focused inflammatory response. However, excessive local and systemic inflammation can be injurious to vital organs near and far from the primary infection site. As the acute injury recedes, pGSN promotes resolution of the inflammatory process and limits the resultant damage.

The possible benefits of adding rhu-pGSN treatment to meropenem were explored in highly lethal, multidrug-resistant *P. aeruginosa* pneumonia in a neutropenic mouse model. All mice died within ~24 hours of infection without immediate antibacterial therapy. Rhu-pGSN as sole treatment slightly prolonged average survival by ~12 hours. To decide on the dose of meropenem that would yield ≥ 50% mortality, titration experiments were performed with each batch of antibiotic. Nonetheless, outcomes were not always predictable, leading to mortality rates ≤ 25% or ≥ 75% for the meropenem controls in some trials. Under such extreme conditions, possible benefits of adjunctive rhu-pGSN on outcome might be masked because the mice were either too sick or not sick enough. Nonetheless, rhu-pGSN given with meropenem was more efficacious than meropenem alone under most conditions.

These preclinical data further strengthen the growing body of evidence that rhu-pGSN as an adjunct to standard-of-care modalities might be effective in enhancing survival while limiting lung injury. Even with supraphysiological levels throughout the dosing interval, neither serious nor drug-related adverse events were observed in rhu-pGSN recipients given three consecutive days of therapy.

Using an established model of murine Gram-negative pneumonia, bacterial colony counts from alveolar lavage and histopathological lung injury scores at the time of euthanasia were higher in mice receiving meropenem alone compared to mice treated with meropenem and rhu-pGSN although there was considerable variability observed within and between experiments. Both mortality and parenchymal injury were lessened by the addition of rhu-pGSN to meropenem, most prominently in situations where meropenem alone was relatively ineffective.

### Example 4 (comparative)

### Methods

### Mouse model of influenza

Normal 6- to 8-week-old male CD1 mice were obtained from Charles River Laboratories (Wilmington, MA). Only male mice were used due to budgetary and time limits. All mice arrived and were co-housed 1 week prior to the start of the experiments. Each trial used a separate batch of mice. A murine-adapted strain of H1N1 influenza virus, A/Puerto Rico/8/1934 (PR8), quantified as plaque-forming units (PFU) was procured from ViraSource (Durham, NC). Mice were anesthetized with 72 mg/kg ketamine plus 9.6 mg/kg xylazine administered via intraperitoneal injection. Mice then received an intranasal instillation of 25 µl suspension of PBS containing virus (ranging from 400-1000 PFU depending on the trial) or vehicle alone. All infections were done at approximately the same time of day (starting at ~10 AM). Initial titration identified 400 PFU as a dose that led to ~60% mortality in vehicle-treated mice, and this dose was used in a majority of the trials (see Figure 12). Most trials used at least 10 mice per group for the vehicle and pGSN treatment groups; details of the influenza dose, total number of mice, and their weights are provided in the tables in Underlying Data [Kobzik L: "Expanded Tables 1 & 2". Harvard Dataverse, V1 2019. www.doi.org/10.7910/DVN/53GJY1].

### Treatments and outcomes

Recombinant human pGSN (rhu-pGSN) was synthesized in E. coli and purified by Fujifilm Diosynth (Billingham, UK). Human rather than murine gelsolin was used based on prior demonstrations of function of rhu-pGSN in rodent models and because data with the human gelsolin will facilitate clinical translation efforts. Rhu-pGSN was administered daily to mice by subcutaneous injection starting on day 3 or 6 after infection, at doses ranging from 0.5-5 mg as detailed in Results. The mice were monitored for 12 days, measuring survival, changes in weight and overall morbidity using a composite index (i.e., 1 point each for hunched appearance, ruffled fur or partly closed eyes; 1.5 points for prolapsed penis or splayed hind quarter; 2 points for listlessness, with a maximum score of 8; the assessment was performed without blinding to treatment group) adapted from guidelines described previously [Burkholder T, et al., Current Protocols Mouse Biol. 2012; 2: 145-65.] Weights and morbidity scores for the last day alive were carried forward for animals that did not survive.

### Lung transcriptome profiling

Lung tissue was obtained on days 7 and 9 after infection from mice treated with either vehicle or rhu-pGSN (dosed 2 mg per day starting on day 3 after infection, then increased to 5 mg per day on day 7). RNA was isolated using the RNAEasy mini-kit (Qiagen, Germantown, MD) according to manufacturer's instructions. RNA samples were analyzed using the Mouse DriverMap targeted gene expression profiling panel from Cellecta (Mountain View, CA). The Cellecta platform uses highly multiplexed RT-PCR amplification and next-generation sequencing (NGS) quantitation to measure expression of 4753 proteincoding and functionally significant mouse genes. The procedure detailed in the Cellecta User Manual, item 5.3 was followed to create amplified index libraries which were sequenced on an Illumina NextSeq 500 instrument. The sequencing data was converted to FASTQ format and then further analyzed using DriverMap Sample Extraction software. This produced a raw data matrix file of counts for each sample in columns aligned to the 4753 gene panel.

### Statistical analysis

Data were analyzed using Prism (GraphPad Software) or SAS (SAS Institute) software. Differences in Kaplan-Meier survival curves were analyzed using a log-rank test with Sidak adjustment for multiple comparisons. A Breslow-Day test for homogeneity of the pGSN versus vehicle comparison across studies yielded p>0.2, indicating homogeneity could not be rejected and supporting the overall comparison across studies, which was carried out via the log-rank (Mantel-Cox) test stratified by trial. For other measurements, differences between groups were examined by ANOVA. The transcriptome profiling results scaled to normalize column counts, were converted to log2 counts (after addition of 0.1 to all cells to eliminate zero values) and then analyzed using Qlucore software (Lund, Sweden). Further analysis of gene set enrichment was performed using tools (Panther version 14.118 and MetaCore (version 19.3, Clarivate Analytics, Philadelphia, PA)) that allow evaluation using a custom background gene list (i.e., the ~4700 genes measured using the Cellecta DriverMap platform).

### Results

### Effect of rhu-pGSN on survival

A variety of dose and timing regimens were tested to evaluate the potential of rhu-pGSN to improve outcomes, conducting a total of 18 trials that are tabulated in Figure 12 and summarized in Figure 13. To mimic likely clinical usage, mice were not treated until several days post-challenge.

A main finding was that delayed treatment with rhu-pGSN resulted in significant improvement in the survival of mice (Fig. 14A-H). All studies combined yielded 39% (93/236) surviving mice treated with vehicle and 62% (241/389) surviving mice treated with pGSN on day 12 (p = 0.000001, Fig. 14A). Improved survival was observed whether the delayed treatment was started on day 6 (Fig. 14C) or day 3 after infection (Fig. 14E, 14G). Similarly, compared to vehicle treatment, rhu-pGSN resulted in decreased morbidity scores (Fig. 14B, 14D, 14F, 14H). In contrast, no statistically significant difference in weight loss or recovery (in surviving animals) was consistently observed in the experiments summarized in Fig. 14A-H. The sole exception was found in the trials testing a dose regimen of initially low (> 2 mg rhu-pGSN on days 3-6/7, then 5 mg through day 11). The latter set of trials led to weights (compared to day 0) at the end of study of 81.4 ± 4.7% in vehicle-treated mice versus 85 ± 2.6% in pGSN-treated mice (p < 0.0001, summary of 4 trials, see also Fig. 12 and Fig. 13, and more detailed tabulation of all experiments in Extended data [Kobzik L: "Expanded Tables 1 & 2". Harvard Dataverse, V1 2019. www.doi.org/10.7910/DVN/53GJY1]. A beneficial effect of rhu-pGSN was observed in a majority but not all of the 18 individual trials (Fig. 12, see Discussion).

### Transcriptome profiling

To evaluate whether rhu-pGSN treatment modified the transcriptome profile [see Harvard Dataverse: Expanded Tables 1 & 2. /doi.org/10.7910/DVN/53GJY 116] of infected lungs, lung tissue was harvested just before (day 7) and after (day 9) the usual onset of mortality (day 8) in this model (n = 5 per group per day). Per protocol, the rhu-pGSN dose was increased in this experiment on day 7, between the 2 time points selected for profiling. Comparison of lung samples obtained at day 7 from vehicle-treated and rhu-pGSN-treated mice showed no significant differences. In contrast, analysis of day 9 samples identified 344 differentially expressed genes in the rhu- pGSN-treated group, comprised of 195 down-regulated and 149 up-regulated genes. The top 50 up- and down-regulated genes are shown in Figure 15, which is notable for the many cytokine and immune-related genes prominent among those down- regulated in the rhu-pGSN-treated group (including IL10, IL12rb, CTLA4, and CCRs9, 7 and 5, among others). Gene enrichment analysis of the full down-regulated gene list was performed using the Panther online analysis tool to query GO Ontology or Reactome databases. The main findings were a reduction of expression of biological processes linked to immune and inflammatory responses, or release of cytokine and other cellular activators. The top 10 most significant processes/pathways are shown in Figure 16. Analysis using a different gene enrichment analysis software tool (MetaCore) produced similar results. Analysis of the up-regulated gene list identified enrichment of processes related to tissue morphogenesis and epithelial/epidermal cell differentiation (consistent with repair of influenza-mediated damage, see Discussion). Details of the DriverMap gene list, the differentially expressed genes identified, and the full results of gene enrichment analyses using the down- and up-regulated gene lists to query the Panther and MetaCore databases are presented in worksheets 2-15 in a spreadsheet available in Extended data [Kobzik L: Harvard Dataverse, V1 2019. www.doi.org/10.7910/DVN/8HBFD7]. Data on experimental groups in studies described herein, are shown in Table 1 and Table 2 of The Harvard Dataverse: Expanded Tables 1 & 2. //doi.org/10.7910/DVN/53GJY116, which also describes additional variables, such as weight, and statistical analyses. Additional data from experiments described herein are provided at: NCBI Gene Expression Omnibus: Transcriptome profiling of lung tissue from influenza-infected mice treated with plasma gelsolin. Accession number GSE138986; //identifiers.org/geo: GSE138986.

### Discussion

Studies were performed to evaluate the potential of rhu-pGSN to improve outcomes in severe influenza using a clinically relevant scenario of delaying initiation of treatment. A key finding was that delayed pGSN treatment significantly improved survival, either when used starting on day 3 or even starting as late as day 6 after infection. In addition to the impractically of initiating earlier therapy right after infection (as opposed to the onset of severe symptoms) in patients, the delay was implemented so as not to interfere with the immediate immune response to influenza given the detrimental consequences observed in some experimental models.

Some limitations merit discussion. The first is the experimental variability observed. Treatment with rhu-pGSN increased survival in a majority of the experiments conducted, but not in all of them. For some of the negative trials, were believed the result of factors such as, but not limited to: technical issues with the virus stock, variation in instillation method, insufficient initial rhu-pGSN dose in the 'low dose then high dose' trials, etc. To the extent possible the methods were adjusted to reduce these potential sources of variability.

Experimental variables were also manipulated, to examine for example, whether treatment as late as day 6 vs day 3 after onset of infection be effective and to assess other variables in the studies. Ultimately, beneficial effects were observed whether the survival analysis included all the trials (Fig. 14A, B) or those using treatment starting at day 6 or day 3 (Fig. 14C-H).

Mice were only followed for 12 days when euthanasia was performed on surviving mice. Because the survival curves were still potentially declining, the ultimate mortality rate could not be confidently ascertained. However, the time to death at a minimum was prolonged with rhu-pGSN over placebo treatment.

Notably, rhu-pGSN did not rescue all of the mice dying from influenza in the experimental model, though the results indicated a significant survival benefit. Given the goal of identifying a novel therapy for severe influenza, it was interpreted that the results obtained in mice without the supportive fluid, additional therapeutic agents (for example but not limited to antiviral agents), and respiratory care given to hospitalized patients, supports a conclusion that methods would confer similar benefits as well as synergistic benefits in clinical settings. The results suggest that combination therapy of administering a gelsolin molecule at a suitable time following infection, with standard therapeutics such as antiviral medications, offers a greater survival advantage.

In summary, rhu-pGSN can improve outcomes in a highly lethal murine influenza model when given after a clinically relevant delay. These findings are consistent with the benefits seen in models of pneumococcal pneumonia. The modes of action for pGSN involve host responses and do not seem to depend on the specific type of pathogen. The experimental results support use of gelsolin as an adjunctive therapy for severe influenza and other viral infections in humans and other mammals.

### Example 5 (comparative)

Additional studies are performed in which synergistic amounts of a gelsolin agent and an antiviral agent. In certain studies oseltamivir phosphate, zanamivir, peramivir or baloxavir marboxil is the antiviral administered to the subject. The gelsolin agent is administered in a delayed-dose method as described above herein. Effective amounts of the antiviral agent and the gelsolin agents are administered to a subject having or suspected of having a viral infection, such as one of Influenza A, B, C, or D and the effective amounts result in a synergistic therapeutic effect against the viral infection in the subject. The synergistic therapeutic effect improves one or more characteristics of the viral infection in the subject by a greater amount than an improvement in the one or more characteristics in a control, wherein the control does not receive a treatment that includes administration of synergistically effective amounts of the gelsolin agent and the antiviral agent.

### Equivalents

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified, unless clearly indicated to the contrary.

The scope of protection is defined in the appended claims.

## Claims

1. A pharmaceutical composition for use in a method of treatment of a bacterial infection resistant to an antibacterial agent in a subject, wherein the bacterial infection is caused by a Pneumococcal species, and wherein:
(a) the pharmaceutical composition comprises a gelsolin molecule, the method comprising administering the pharmaceutical composition to the subject and administering the antibacterial agent to which the bacterial infection is resistant to the subject, to thereby treat the bacterial infection, wherein the gelsolin molecule and the antimicrobial agent are administered in the same or separate formulations, but are administered to be in the subject at the same time; or
(b) the pharmaceutical composition comprises the antibacterial agent to which the bacterial infection is resistant, the method comprising administering the pharmaceutical composition to the subject and administering a gelsolin molecule to the subject, to thereby treat the bacterial infection wherein the gelsolin molecule and the antimicrobial agent are administered in the same or separate formulations, but are administered to be in the subject at the same time; or
(c) the pharmaceutical composition comprises a gelsolin molecule and the antibacterial agent to which the bacterial infection is resistant, the method comprising administering the pharmaceutical composition to the subject, to thereby treat the bacterial infection;
wherein the gelsolin molecule is a plasma gelsolin (pGSN) or a cytoplasmic gelsolin (cGSN), and wherein the antibacterial agent comprises a β-lactam antibiotic.

2. The pharmaceutical composition for use in a method of treatment of claim 1, wherein the gelsolin molecule is plasma gelsolin (pGSN), and optionally is a recombinant pGSN, and optionally is a recombinant human pGSN.

3. The pharmaceutical composition for use in a method of treatment of claim 1, wherein the antibacterial agent comprises penicillin.

4. The pharmaceutical composition for use in a method of treatment of claim 1, wherein the amount of the antibacterial-agent is an amount below a minimum effective dose (MED) of the antibacterial-agent in the subject, wherein the MED of the antibacterial-agent is a lowest dose level of the antibacterial-agent that provides a clinically significant response in average efficacy, wherein the response is statistically significantly greater than a response provided by a control that does not include the dose of the antibacterial-agent.

5. The pharmaceutical composition for use in a method of treatment of claim 1, wherein the subject is a mammal.

6. The pharmaceutical composition for use in a method of treatment of claim 1, wherein the subject is a human.

## Patentansprüche

1. Pharmazeutische Zusammensetzung zur Verwendung bei einem Verfahren zur Behandlung einer gegenüber einem antibakteriellen Mittel resistenten bakteriellen Infektion bei einem Subjekt, wobei die bakterielle Infektion durch eine Pneumokokken-Spezies verursacht wird und wobei:
(a) die pharmazeutische Zusammensetzung ein Gelsolin-Molekül umfasst, wobei das Verfahren Verabreichen der pharmazeutischen Zusammensetzung an das Subjekt und Verabreichen des antibakteriellen Mittels, gegenüber dem die bakterielle Infektion resistent ist, an das Subjekt umfasst, um dadurch die bakterielle Infektion zu behandeln, wobei das Gelsolin-Molekül und das antimikrobielle Mittel in den gleichen oder separaten Formulierungen verabreicht werden, aber so verabreicht werden, dass sie sich gleichzeitig in dem Subjekt befinden; oder
(b) die pharmazeutische Zusammensetzung das antibakterielle Mittel, gegenüber dem die bakterielle Infektion resistent ist, umfasst, wobei das Verfahren Verabreichen der pharmazeutischen Zusammensetzung an das Subjekt und Verabreichen eines Gelsolin-Moleküls an das Subjekt umfasst, um dadurch die bakterielle Infektion zu behandeln, wobei das Gelsolin-Molekül und das antimikrobielle Mittel in den gleichen oder separaten Formulierungen verabreicht werden, aber so verabreicht werden, dass sie sich gleichzeitig in dem Subjekt befinden; oder
(c) die pharmazeutische Zusammensetzung ein Gelsolin-Molekül und das antibakterielle Mittel, gegenüber dem die bakterielle Infektion resistent ist, umfasst, wobei das Verfahren Verabreichen der pharmazeutischen Zusammensetzung an das Subjekt umfasst, um dadurch die bakterielle Infektion zu behandeln;
wobei das Gelsolin-Molekül ein Plasma-Gelsolin (pGSN) oder ein zytoplasmatisches Gelsolin (cGSN) ist und wobei das antibakterielle Mittel ein β-Lactam-Antibiotikum umfasst.

2. Pharmazeutische Zusammensetzung zur Verwendung bei einem Verfahren zur Behandlung nach Anspruch 1, wobei das Gelsolin-Molekül Plasma-Gelsolin (pGSN) ist und optional ein rekombinantes pGSN ist und optional ein rekombinantes humanes pGSN ist.

3. Pharmazeutische Zusammensetzung zur Verwendung bei einem Verfahren zur Behandlung nach Anspruch 1, wobei das antibakterielle Mittel Penicillin umfasst.

4. Pharmazeutische Zusammensetzung zur Verwendung bei einem Verfahren zur Behandlung nach Anspruch 1, wobei die Menge des antibakteriellen Mittels eine Menge unter einer minimalen wirksamen Dosis (MED) des antibakteriellen Mittels bei dem Subjekt ist, wobei die MED des antibakteriellen Mittels ein niedrigstes Dosisniveau des antibakteriellen Mittels ist, das ein klinisch signifikantes Ansprechen mit durchschnittlicher Wirksamkeit bereitstellt, wobei das Ansprechen statistisch signifikant größer ist als ein Ansprechen, das durch eine Kontrolle bereitgestellt wird, die die Dosis des antibakteriellen Mittels nicht beinhaltet.

5. Pharmazeutische Zusammensetzung zur Verwendung bei einem Verfahren zur Behandlung nach Anspruch 1, wobei das Subjekt ein Säugetier ist.

6. Pharmazeutische Zusammensetzung zur Verwendung bei einem Verfahren zur Behandlung nach Anspruch 1, wobei das Subjekt ein Mensch ist.

## Revendications

1. Composition pharmaceutique pour l'utilisation dans un procédé de traitement d'une infection bactérienne résistante à un agent antibactérien chez un sujet, dans laquelle l'infection bactérienne est causée par une espèce de pneumocoque, et dans laquelle :
(a) la composition pharmaceutique comprend une molécule de gelsoline, le procédé comprenant l'administration de la composition pharmaceutique au sujet et l'administration de l'agent antibactérien auquel l'infection bactérienne est résistante au sujet, pour le traitement de ce fait de l'infection bactérienne, dans laquelle la molécule de gelsoline et l'agent antimicrobien sont administrés dans la même formulation ou dans des formulations séparées, mais sont administrés pour la présence chez le sujet en même temps ; ou
(b) la composition pharmaceutique comprend l'agent antibactérien auquel l'infection bactérienne est résistante, le procédé comprenant l'administration de la composition pharmaceutique au sujet et l'administration d'une molécule de gelsoline au sujet, pour le traitement de ce fait de l'infection bactérienne, dans laquelle la molécule de gelsoline et l'agent antimicrobien sont administrés dans la même formulation ou dans des formulations séparées, mais sont administrés pour la présence chez le sujet en même temps ; ou
(c) la composition pharmaceutique comprend une molécule de gelsoline et l'agent antibactérien auquel l'infection bactérienne est résistante, le procédé comprenant l'administration de la composition pharmaceutique au sujet, pour le traitement de ce fait de l'infection bactérienne ;
dans laquelle la molécule de gelsoline est une gelsoline plasmatique (pGSN) ou une gelsoline cytoplasmique (cGSN), et dans laquelle l'agent antibactérien comprend un antibiotique β-lactame.

2. Composition pharmaceutique pour l'utilisation dans un procédé de traitement selon la revendication 1, dans laquelle la molécule de gelsoline est la gelsoline plasmatique (pGSN), et éventuellement est une pGSN recombinante, et éventuellement est une pGSN humaine recombinante.

3. Composition pharmaceutique pour l'utilisation dans un procédé de traitement selon la revendication 1, dans laquelle l'agent antibactérien comprend de la pénicilline.

4. Composition pharmaceutique pour l'utilisation dans un procédé de traitement selon la revendication 1, dans laquelle la quantité de l'agent antibactérien est une quantité en dessous d'une dose efficace minimale (MED) de l'agent antibactérien chez le sujet, dans laquelle la MED de l'agent antibactérien est le niveau de dose le plus bas de l'agent antibactérien qui fournit une réponse cliniquement significative en efficacité moyenne, dans laquelle la réponse est statistiquement significativement plus grande qu'une réponse fournie par un témoin qui n'inclut pas la dose de l'agent antibactérien.

5. Composition pharmaceutique pour l'utilisation dans un procédé de traitement selon la revendication 1, dans laquelle le sujet est un mammifère.

6. Composition pharmaceutique pour l'utilisation dans un procédé de traitement selon la revendication 1, dans laquelle le sujet est un humain.
